# EUROPEAN PATENT SPECIFICATION

(11) **EP 2 131 879 B1**
(45) Date of publication and mention of the grant of the patent: **09.10.2019**
(21) Application number: 08732144.4
(22) Date of filing: 13.03.2008
(51) Int. Cl.: A61L 27/14, A61L 27/50, A61L 31/04, A61L 31/14

(54) **INTERNAL FIXATION DEVICES**
INTERNE FIXIERUNGSVORRICHTUNGEN
DISPOSITIFS DE FIXATION INTERNES

(30) Priority: 13.03.2007 US 894505 P; 19.04.2007 US 912845 P; 19.04.2007 US 912738 P; 19.04.2007 US 912740 P; 19.11.2007 US 989113 P
(43) Date of publication of application: 16.12.2009
(73) Proprietor: Smith & Nephew, Inc., Memphis, TN 38116 (US)
(72) Inventor: AUSTIN, Gene Edward, Bartlett, Tennessee 38135 (US); BETTENGA, Mason, Memphis, Tennessee 38122 (US); BROWN, Malcolm, Otley Yorkshire (GB); BRUMFIELD, David L., Collierville, TN 38017 (US); EVANS, David L., Bartlett, Tennessee 38135 (US); FABER, Henry B., Memphis, TN 38120 (US); FARRAR, David, Fulford Yorkshire Y01 4OG (GB); HALL, Michael, Linthorpe Yorkshire (GB); MONTES DE OCA BALDERAS, Horacio, York Yorkshire (GB); RAINS, James K., Cordova, Tennessee 38016 (US); ROSE, John, Collierville, Tennessee 38017 (US)
(74) Representative: Smith & Nephew
(86) International application number: PCT/US2008/056882
(87) International publication number: WO 2008/112912

(56) References cited:
- EP-A- 0 360 139
- EP-A- 1 000 958
- WO-A-01/54598
- WO-A-2006/116164
- DE-A1- 3 417 923
- US-A- 6 127 597

## Description

This application is a PCT International Application claiming priority to United States Patent Application No. 60/894,505 filed on March 13, 2007, United States Patent Application No. 60/912,845 filed on April 19, 2007, United States Patent Application No. 60/912,738 filed on April 19, 2007, United States Patent Application No. 60/912,740 filed on April 19, 2007, and United States Patent Application No. 60/989,113 filed on November 19, 2007.

### BACKGROUND OF THE INVENTION

### FIELD OF THE INVENTION

The present invention relates to internal fixation devices for use in bone fracture repair and more specifically, an internal fixation devices that include a polymer material for improved device stabilization and fracture fixation.

### RELATED ART

Problems can arise when a bone fracture or fusion site is not sufficiently stabilized during the healing lifetime. Depending on the nature of the fracture, internal fixation devices, such as intramedullary nails and screws, may be used alone, or in combination. One goal of these devices is the anatomic reduction of the fracture. Another goal is to minimize or eliminate interfragmentary motion. Still another goal involves increasing or maximizing blood supply to the fracture site by reducing or minimizing additional vascular damage. Sustained compressive therapy can also be osteoinductive, due to its piezoelectric effects on osteoblasts themselves. Excessive interfragmentary motion results in the formation of fibrous, unmineralized scar tissue (resulting in non-union or pseudo-arthrosis) versus regeneration of bone. The unmineralized scar tissue is not load supporting and skeletal function is lost. A sufficient blood supply must be maintained to support skeletal metabolism, bone regeneration, and remodeling of the fracture site.

These internal fixation devices are made of metal, such as stainless steel or titanium. Overtime, however, these stainless steel and titanium fixation devices do not maintain adequate fixation to bone or compression across the fracture fragments. As the necrotic surfaces of the fracture are resorbed, a non-load bearing gap develops between the fragments, thereby decreasing compression and increasing the risk of interfragmentary motion and scar tissue formation. Loss of compression is contrary to the objectives of fracture fixation in general and these devices in particular. Improvements are therefore desired to help improve fixation to bone and maintain compressive load across the fracture site over a longer period of healing.

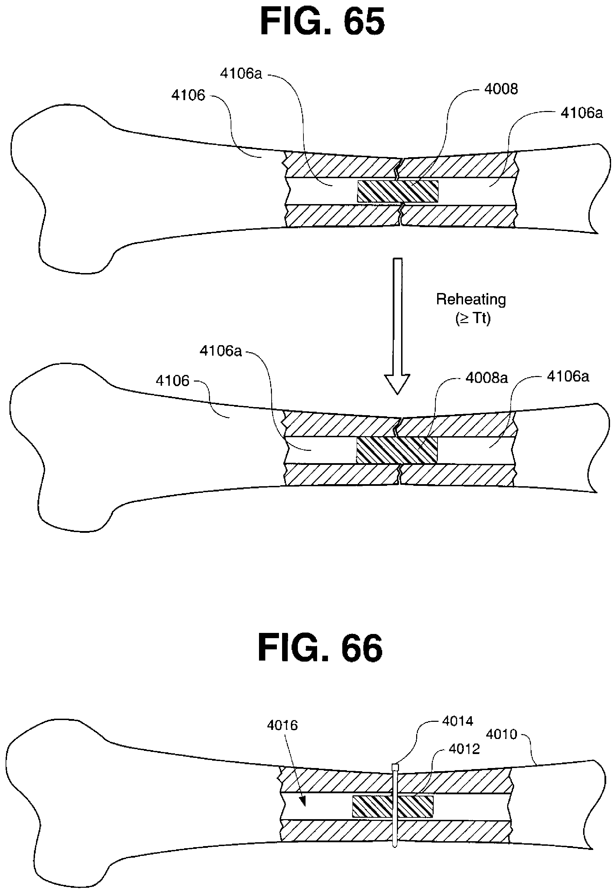

U.S. Patent No. 6,281,262 issued on Aug. 28, 2001 discloses use of a shape memory polymer for bone fixation. The shape memory polymer for bone fixation is illustrated in FIG. 65. This shape-memory material for bone fixation (4008) is a molded article made of a lactic acid-based polymer in the shape of bars. When it is heated to a deformation temperature (i.e., the deformation temperature (Tf) as will be described hereinafter), it can be recovered to the memorized shape of thick and short bars, compared with those before reheating, without applying any external force thereto. This shape-memory material for bone fixation (4008) is prepared by compressing and deforming a molded article made of a lactic acid-based polymer in the shape of thick and round bars into another molded article in the shape of round bars longer and thinner than said ones at a deformation temperature higher than the glass transition temperature (Tg) thereof but lower than the crystallization temperature (Tc) thereof and then fixing the molded article to the shape of thin and round bars by cooling it as such to a temperature lower than the glass transition temperature (Tg).

When this shape-memory material for bone fixation (4008) is heated to the deformation temperature (Tf) or above, it is immediately recovered to the original molded article in the shape of thick and round bars. As FIG. 65 shows, therefore, this shape-memory material for bone fixation (4008) is used as a substitute for conventional intramedullary nails. Namely, the shape-memory material (4008) is inserted equally into the intramedullary canal (4106a, 4106a) of both sections (4106, 4106) of a broken or incised bone. Then this shape-memory material is reheated by, for example, bringing into contact with hot water (sterilized saline) at the deformation temperature (Tf) or above. As a result, the shape-memory material for bone fixation (4008) is recovered to the original molded article (4008a) in the shape of a thick and round bar and comes in contact closely to the endosteal surface and/or cancellous bone of the intramedullary canal (4106a, 4106a). Namely, the shape-memory material (4008) is fixed tightly, and the bone sections (4106, 4106) can be easily and surely fixed together.

The '262 Patent assumes that the shape memory material will expand uniformly and remain positioned relative to the fracture site. However, in practice, this is not always the case. For example, the shape memory material may expand more quickly on one side of the fracture site or another. In other words, the material may shift significantly to one side of the fracture or the other. This may cause the shape memory polymer to inadequately support one bone section or the other.

Further, the '262 Patent assumes that it always desirable to place the center of the shape memory material relative to the fracture site. This may not always be the case. For example, if the fracture site is located proximate to the end of a bone, it would be desirable to achieve adequate expansion on each side of the fracture site even though more material may be located on one side of the fracture than the other.

### SUMMARY OF THE INVENTION

In one aspect, the present disclosure relates to an internal fixation device including an interface portion and a polymer material coupled to the interface portion, wherein the polymer material includes at least one feature on a surface of the polymer material. In an embodiment, the polymer material includes multiple features. In another embodiment, the feature includes a particulate material. In another embodiment, the particulate material includes a ceramic material. In yet another embodiment, the feature includes a protrusion. In a further embodiment, the protrusion is selected from a group including a metal material, a non-metal material, a polymer material, and combinations thereof. In yet a further embodiment, the polymer material of the internal fixation device and the protrusion includes a resorbable material or a non-resorbable material. In still yet a further embodiment, the polymer material of the fixation device and the protrusion includes shape memory qualities.

In another aspect, the present disclosure relates to a method of fixating an internal fixation device to a bone. The method includes providing an internal fixation device having an interface portion and a polymer material coupled to the interface portion, wherein the polymer material includes at least one feature on a surface of the polymer material; inserting the internal fixation device into a bone; and providing the polymer material with energy to deform the material and fixate the internal fixation device to the bone.

In a further aspect, the present disclosure relates to an internal fixation device including a channel and a shape memory polymer material located within the channel. In an embodiment, the channel partially extends a length of the device. In another embodiment, the shape memory polymer material includes a body having a stem portion, wherein the stem portion is located within the channel. In yet another embodiment, the internal fixation device includes a proximal portion and a distal portion, the shape memory polymer material located at the distal portion. In a further embodiment, the distal portion includes a hinge. In a further embodiment, the distal portion includes at least one feature on a surface of the distal portion. In yet a further embodiment, the feature includes a protrusion.

In yet a further aspect, the present disclosure relates to a method of fixating an internal fixation device to a bone including providing an internal fixation device including a channel and a shape memory polymer material located within the channel; inserting the internal fixation device into a bone; and providing the polymer material with energy to deform the material and fixate the internal fixation device to the bone. In an embodiment, the internal fixation device includes a proximal portion and a hinged distal portion, the shape memory polymer material located at the distal portion. In another embodiment, the distal portion extends outward and engages in the bone when the polymer material is provided with energy.

In yet a further aspect, the present disclosure relates to an internal fixation device including a cannulated inner portion, an outer portion, at least two C-shaped channels located on the outer portion, the channels located on opposite sides of the device from each other, wherein each channel includes a tab, and a polymer material, the polymer material located within the cannulated inner portion and between the C-shaped channels.

In an even further aspect, the present disclosure relates to a method of fixating an internal fixation device to a bone including providing an internal fixation device including a cannulated inner portion, an outer portion, at least two C-shaped channels located on the outer portion, the channels located on opposite sides of the device from each other, wherein each channel includes a tab, and a polymer material, the polymer material located within the cannulated inner portion and between the C-shaped channels; inserting the internal fixation device into a bone; and providing the polymer material with energy to deform the material, wherein deforming the material causes the tabs to open and engage in the bone to fixate the device.

There is provided a fracture fixation device that allows for adequate expansion on each side of a fracture site. The fracture fixation device achieves desired placement of an expanded shape memory material.

In some embodiments, the fracture fixation device achieves symmetrical fixation such that the general center of the shape memory material remains generally stationary relative to the fracture site as the shape memory material shortens.

In other embodiments, the fracture fixation device achieves asymmetrical fixation such that the shape memory material adequately expands on each side of the fracture site but each bone section has a different amount of shape memory material than the other.

In some embodiments, the shortening of the shape memory material may be used to achieve compression of the fracture.

In some embodiments, the shape memory material may be cannulated.

In some embodiments, the shape memory material may be radio-opaque.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The accompanying drawings, which are incorporated in and form a part of the specification, illustrate the embodiments of the present invention and together with the written description serve to explain the principles, characteristics, and features of the invention. In the drawings:
Fig. 1 shows a perspective view of an internal fixation device of the present disclosure.
Fig. 2A shows a cross-sectional view of an interface portion having a circular shape.
Fig. 2B shows a cross-sectional view of an interface portion having a triangular shape.
Fig. 2C shows a cross-sectional view of an interface portion having a rectangular shape.
Fig. 2D shows a cross-sectional view of an interface portion having a star shape.
Fig. 2E shows a cross-sectional view of an interface portion having an oval shape.
Fig. 2F shows a cross-sectional view of an interface portion having a hexagonal shape.
Fig. 2G shows a cross-sectional view of an interface portion having a Chinese star shape.
Fig. 2H shows a perspective view of an interface portion having a tapered surface.
Fig. 2I shows a perspective view of an interface portion having a beveled surface.
Fig. 2J shows a perspective view of an interface portion having a surface with axial and radial grooves.
Fig. 2K shows a perspective view of an interface portion having a surface with helical grooves.
Fig. 2L shows a perspective view of a sleeve of polymer material for use on a shaped interface portion of an internal fixation device of the present disclosure.
Fig. 2M shows a perspective view of a shaped interface portion including strips of polymer material.
Fig. 3 shows a perspective view of an internal fixation device of the present disclosure having multiple interface portions.
Fig. 4 shows a perspective view of a second internal fixation device of the present disclosure.
Fig. 5 shows a first method of fixating an internal fixation device to a bone.
Figs. 6A and 6B illustrate an embodiment of internal fixation of the first method.
Fig. 7 shows a second method of fixating an internal fixation device to a bone.
Fig. 8 illustrates a first embodiment of internal fixation of the second method.
Fig. 9 illustrates a second embodiment of internal fixation of the second method.
Fig. 10 illustrates a third embodiment of internal fixation of the second method.
Fig. 11 shows a third method of fixating an internal fixation device to a bone.
Fig. 12 shows a first embodiment of internal fixation of the third method.
Fig. 13 shows a second embodiment of internal fixation of the third method.
Fig. 14 shows a method of stabilizing a fractured bone.
Figs. 15A and 15B illustrate a first embodiment of the fracture stabilization method.
Figs. 16A and 16B illustrate a second embodiment of the fracture stabilization method.
Fig. 17 shows a perspective view of an internal fixation device of the present disclosure having an interface portion that includes one hole.
Fig. 18 shows a perspective view of an internal fixation device of the present disclosure having an interface portion that includes multiple holes.
Fig. 19 shows a perspective view of an internal fixation device of the present disclosure having an interface portion that includes screw threads.
Fig. 20 shows a perspective view of an internal fixation device of the present disclosure having an interface portion that includes circumferential ribs.
Figs 21A and 21B show a perspective view of an internal fixation device of the present disclosure having interface portions that include engravings.
Fig. 22 shows a perspective view of an internal fixation device of the present disclosure having multiple interface portions.
Fig. 23 shows a perspective view of a sleeve of polymer material for use on a shaped interface portion of an internal fixation device of the present disclosure.
Fig. 24 shows a perspective view of an interface portion of the present disclosure having engravings that include strips of polymer material.
Fig. 25A shows a perspective view of an internal fixation device of the present disclosure.
Fig. 25B shows a perspective view of an internal fixation device of the present disclosure.
Fig. 26 shows a method of fixating an internal fixation device to a bone.
Figs. 27A and 27B illustrate an embodiment of an internal fixation of the method of Fig. 26.
Fig. 28 shows a method of fixating an internal fixation device to a bone.
Fig. 29 illustrates a first embodiment of an internal fixation of the method of Fig. 28.
Fig. 30 illustrates a second embodiment of an internal fixation of the method of Fig. 28.
Fig. 31 illustrates a third embodiment of an internal fixation of the method of Fig. 28.
Fig. 32 shows a method of fixating an internal fixation device to a bone.
Fig. 33 shows a first embodiment of an internal fixation of the method of Fig. 32.
Fig. 34 shows a second embodiment of an internal fixation of the method Fig. 32.
Fig. 35 shows a first method of stabilizing a fractured bone.
Figs. 36A and 36B illustrate a first embodiment of the fracture stabilization method of Fig. 35.
Figs. 37A and 37B illustrate a second embodiment of the fracture stabilization method of Fig. 35.
Fig. 38 shows a method of stabilizing a fractured bone.
Figs. 39A and 39B illustrate an embodiment of fracture stabilization of the method of Fig. 38.
Figs. 40A and 40B show a perspective view of an internal fixation device of the present disclosure before and after deformation of the polymer material.
Fig. 41A shows a cross-sectional view of a fastener after insertion of the fastener into a hole having a polymer material and prior to deformation of the material.
Fig. 41B shows a cross-sectional view of a fastener after insertion of the fastener into a hole having a polymer material and after deformation of the polymer material.
Figs. 42A and 42B illustrate use of the internal fixation device of Figs. 40A and 40B for fracture stabilization.
Figs. 43A and 43B show a perspective view of the internal fixation device of the present disclosure before and after deformation of the polymer material.
Figs. 44A and 44B illustrate use of the internal fixation device of Figs. 43a and 43B for fracture stabilization.
Figs 45A and 45B show a side view of an internal fixation device of the present disclosure before and after deformation of the polymer material.
Fig. 45C shows a top view of the internal fixation device of Figs. 45A-45B.
Figs. 46A-46B illustrate use of the internal fixation device of Figs. 45A-45B for fracture stabilization.
Figs. 47A and 47B illustrate use of an internal fixation device and a washer to stabilize a fracture.
Fig. 48 shows a perspective view of an internal fixation device of the present disclosure.
Figs. 49A and 49B illustrate a first embodiment of us of an internal fixation device of Fig. 48.
Figs. 50A and 50B illustrate a second embodiment of use of an internal fixation device of Fig. 48.
Figs. 51A and 51B show a perspective view of an internal fixation device of the present disclosure before and after deformation of the polymer material.
Figs. 52A and 52B show a cross-sectional view of an internal fixation device of the present disclosure before and after deformation of the polymer material.
Figs. 53A and 53B show a cross-sectional view of an internal fixation device of the present disclosure before and after deformation of the polymer material.
Figs. 54A and 54B show a perspective view of an internal fixation device of the present disclosure before and after deformation of the polymer material.
Fig. 55A shows a perspective view of a fastener, having a head that includes a shape memory polymer material, after insertion of the fastener into a hole and prior to deformation of the material.
Fig. 55B shows a perspective view of a fastener, having a head that includes a shape memory polymer material, after insertion of the fastener into a hole and after deformation of the polymer material.
Figs. 56A and 56B illustrate an embodiment of fracture stabilization.
Figs. 57A and 57B show a cross-sectional view of an internal fixation device of the present disclosure before and after deformation of the polymer material.
Fig. 58A shows a perspective view of an internal fixation device of the present disclosure.
Figs. 58B and 58C show top cross-sectional views of the C-shaped channel region of the internal fixation device of Fig. 58A before and after deformation of the polymer material.
Fig. 59 shows a method of fixating a plate to a fractured bone.
Fig. 60A shows a perspective view of an internal fixation device of the present disclosure.
Fig. 60B illustrates use of the internal fixation device of Fig. 60A in fracture fixation.
Figs. 61A-61B show cross-sectional views of an internal fixation device of the present disclosure before and after deformation of a shape memory polymer material.
Figs. 62A-62B show cross-sectional end views of an internal fixation device located in bone.
Fig. 63 shows pullout test results for two embodiments of the internal fixation device of the present disclosure.
Fig. 64 shows torque test results for two embodiments of the internal fixation device of the present disclosure.
FIG. 65 illustrates a bone fixation device as disclosed in the prior art.
FIG. 66 illustrates a fastener for locating a shape memory material.
FIG. 67 illustrates a plurality of fasteners for locating a shape memory material.
FIG. 68 illustrates a cut-to-length shape memory material.
FIG. 69 illustrates a first and a second cross section of a shape memory material.
FIG. 70A illustrates a heating device having a plurality of heating elements.
FIG. 70B illustrates a heating device having a plurality of insulation elements.
FIG. 71 illustrates a first embodiment of a shape memory material having at least two glass transition temperatures.
FIG. 72 illustrates a second embodiment of a shape memory material having at least two glass transition temperatures.
FIG. 73 illustrates an implant assembly.
FIG. 74 illustrates a first instrument for placement of the shape memory material.
FIG. 75 illustrates a second instrument for placement of the shape memory material.
FIG. 76 illustrates a third instrument for placement of the shape memory material.
FIG. 77 illustrates a shape memory material in a first embodiment.
FIG. 78 illustrates a shape memory material in a second embodiment.
FIG. 79 illustrates a shape memory material in a third embodiment.
FIG. 80 illustrates a shape memory material in a fourth embodiment.
FIG. 81 illustrates a shape memory material in a fifth embodiment.
FIG. 82 illustrates a fourth instrument for placement of the shape memory material.
FIG. 83 illustrates the installed shape memory material.
FIG. 84 illustrates a shapable reamer.
FIG. 85 illustrates a bone after reaming using the shapable reamer.
FIG. 86 illustrates the shape memory material as installed in the reamed intramedullary cavity.
FIG. 87 illustrates an exemplary embodiment of the shapable reamer.
FIG. 88 illustrates a first embodiment of a balloon compression device.
FIG. 89 illustrates a second embodiment of a balloon compression device.
FIG. 90 illustrates a method for manufacturing a shape memory material having at least two glass transition temperatures.
FIG. 91 illustrates a tapered heater in a first embodiment.
FIG. 92 illustrates the shape memory polymer after heating using the tapered heater of FIG. 91.
FIG. 93 illustrates a tapered heater in a second embodiment.
FIG. 94 illustrates the shape memory polymer after heating using the tapered heater of FIG. 93.

### DETAILED DESCRIPTION OF THE EMBODIMENTS

The following description of the preferred embodiment(s) is merely exemplary in nature and is in no way intended to limit the invention, its application, or uses.

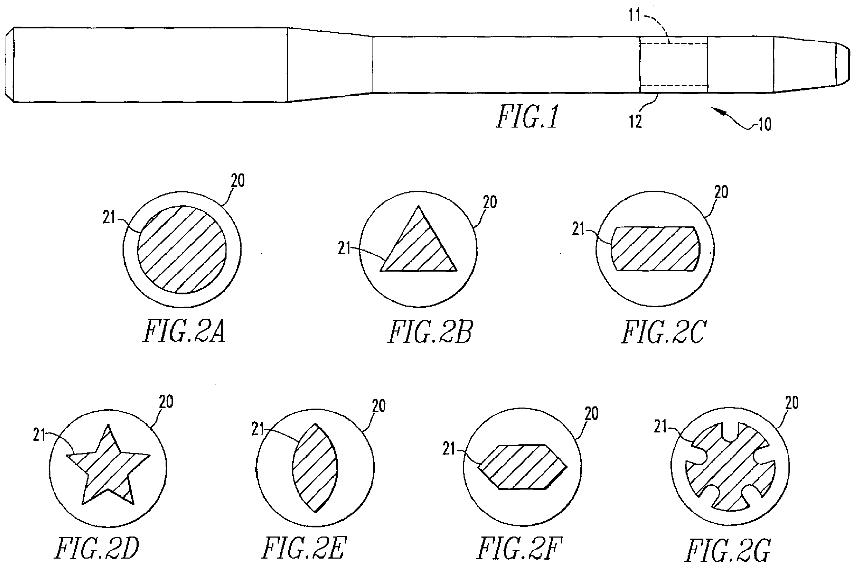

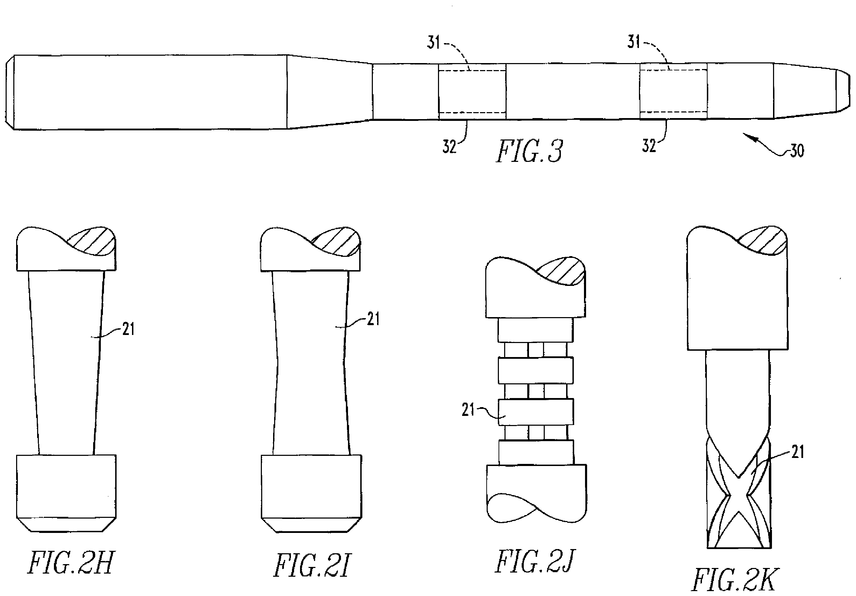

Fig. 1 shows an internal fixation device **10** including a shaped interface portion **11** and a polymer material **12** coupled to the shaped interface portion **11.** The internal fixation device **10** is an intramedullary nail, but could be any other internal fixation device that is used in the repair of bone fractures, such as a bone screw, a locking screw, or a rod. The shaped interface portion **11** has a square shape, but can be of any other shape that allows formation of bonds between the polymer material **12** and the shaped interface portion **11** once the polymer material is provided with energy, as described below. As shown in Figs. 2A-2G, the shaped interface portion **21** may include a shape that is circular, triangular, rectangular, star-shaped, oval, hexagonal, or Chinese star shaped, respectively. In addition, as also shown in Figs. 2H-2K, the surface of the shaped interface portion **21** may be tapered or beveled or include axial and/or radial grooves or helical grooves, respectively. These shapes and surfaces help the polymer material engage the device to provide support for axial and torsional loading and to substantially reduce motion in those directions after the device has been placed in a bone, as will be further described below. The shapes and surfaces can be machined, molded, cast, laser cut, or chemically etched into the internal fixation device or formed via another method known to one of ordinary skill in the art. Machining of the shapes and surfaces could take many forms, including wire and ram electrical discharge machining (EDM). In addition, the shaped interface portion may be located anywhere along the device.

As shown in Fig. 3, multiple shaped interface portions **31,** including a polymer material **32,** may be present on the internal fixation device **30** and the portions **31** may include a surface and a shape having a cross-section as described above. In addition, the shaped interface portions **31** may be present anywhere along the internal fixation device **30.**

The polymer material that is coupled to the shaped interface portion includes an orientated resorbable or non-resorbable material and is selected from a group that includes an amorphous polymer, a semi-crystalline polymer, or a composition having a combination thereof. The polymer material may also include a shape memory polymer. Factors used to determine the type of polymer used on the shaped interface portion, include, but are not limited to, the desired amount of polymer deformation, the desired rate at which that deformation occurs, the rate at which the polymer is absorbed, and the strength of the polymer.

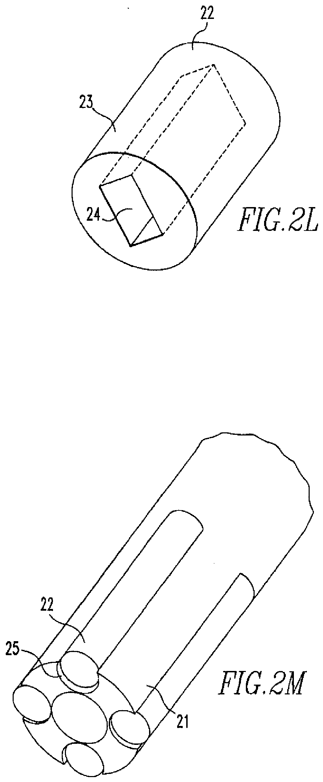

The orientated polymer material could include a sleeve of material having a uniform structure with an outside surface and a channel running through the middle of the structure with both the structure and the channel having the same or different shapes. For the purposes of Figs. 1 & 3 and as shown in Fig. 2L, the polymer material is in the form of a sleeve **22** having a cylindrical structure with an outside surface **23** that is circular and a channel **24** having a square shape to match the square shape of the shaped interface portion. However, the structure of the sleeve **22** and the channel **24** may have another shape. The sleeve **22** may be formed by die-drawing or molding (i.e. compression flow molding or thermoforming process) the above-mentioned polymers or polymer compositions. The channel **24** may be formed in the sleeve **22** during the die drawing or molding process. Alternatively, the channel **22** may be formed in the sleeve **22** post processing by drilling or by any other method of forming the channel **22.**

In addition, the polymer material may not be in the form of sleeve, but rather there may be several strips of polymer material each of which have a structure and each of which are coupled to the shaped interface portion. For example, a shaped interface portion **21** having a Chinese star shape, such as in Fig. 2M, would have strips of polymer material **22** coupled to the slotted areas **25** of the shaped interface portion **21.** However, the polymer material may be in other forms. The strips **22** may be formed by the processes listed above or by another process, such as an extrusion process (i.e. single screw, twin screw, disk, ram, or pultrusion process).

Furthermore, for the purposes of this disclosure, the outer surface of the polymer material is shown, in Figs. 1, 3, 4, 15A, and 16A, as being flush, or forming the same plane with, the outer surface of the fixation device. However, the outer surface of the polymer material may be of a smaller or larger diameter than the outer surface of the fixation device.

The internal fixation device may be manufactured from a metal, such as titanium, titanium alloys, steel, stainless steel, cobalt-chromium alloys, tantalum, magnesium, niobium, nickel, nitinol, platinum, silver, and combinations thereof. Other metals known to one of ordinary skill in the art could also be used. The device may also be manufactured from a resorbable or non-resorbable polymer material and may be the same polymer material used on the shaped interface portion, as described above, or another type of polymer material.

Specific polymers that may be used for the shaped interface portion and/or the device include polyetheretherketone (PEEK), polymethyl methacrylate (PMMA), polyethyl methacrylate (PEMA), polyacrylate, poly-alpha-hydroxy acids, polycapropactones, polydioxanones, polyesters, polyglycolic acid, polyglycols, polylactides, polyorthoesters, polyphosphates, polyoxaesters, polyphosphoesters, polyphosphonates, polysaccharides, polytyrosine carbonates, polyurethanes, and copolymers or polymer blends thereof. In addition, bioactive agents may be incorporated into the polymer material to be released during the deformation or the degradation of the polymer material. These agents are included to help promote bone regrowth. Examples include bone morphogenic proteins, antibiotics, anti-inflamatoies, angiogenic factors, osteogenic factors, monobutyrin, omental extracts, thrombin, modified proteins, platelet rich plasma/solution, platelet poor plasma/solution, bone marrow aspirate, and any cells sourced from flora or fawna, such as living cells, preserved cells, dormant cells, and dead cells. Other bioactive agents known to one of ordinary skill in the art may also be used. Furthermore, the polymeric materials can be formed as a composite or matrix and include reinforcing material or phases such as fibers, rods, platelets, and fillers. For example, the polymeric material can include glass fibers, carbon fibers, polymeric fibers, ceramic fibers, or ceramic particulates. Other reinforcing material or phases known to one of ordinary skill in the art could also be used.

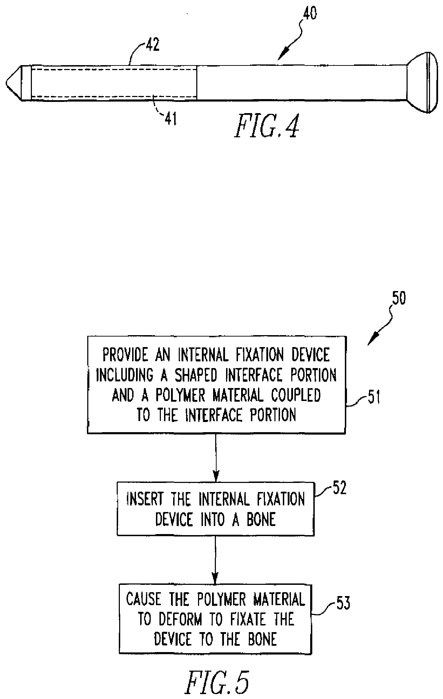

Fig. 4 shows another example of an internal fixation device **40** that includes a shaped interface portion **41** and a polymer material **42** coupled to the interface portion **41.** The internal fixation device **40** of Fig. 4 includes a bone screw or locking screw. The physical and compositional properties of the intramedullary nail, shaped interface portion, and polymer material, as described above, also apply to the screw in Fig. 4.

Fig. 5 shows a first method of fixating an internal fixation device to a bone **50.** An internal fixation device is provided that includes a shaped interface portion and a polymer material coupled to the interface portion **51.** The internal fixation device is then inserted into a bone **52** and the polymer material is caused to deform **53,** thereby fixating the internal fixation device to the bone.

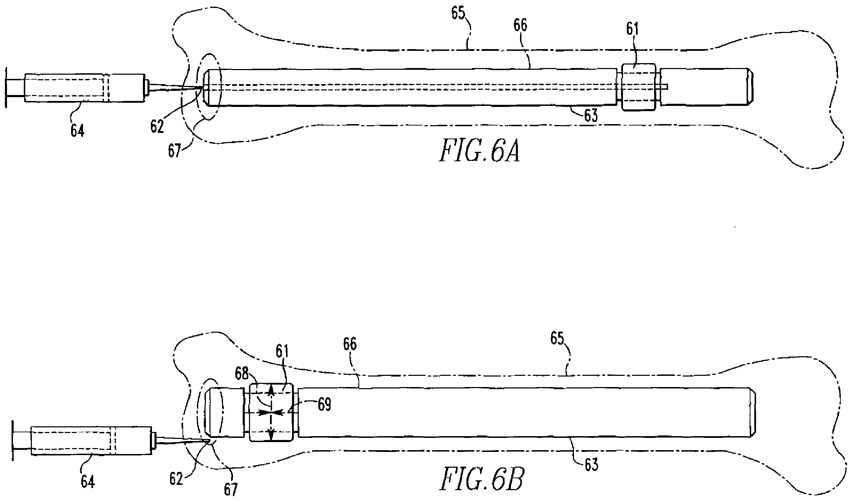

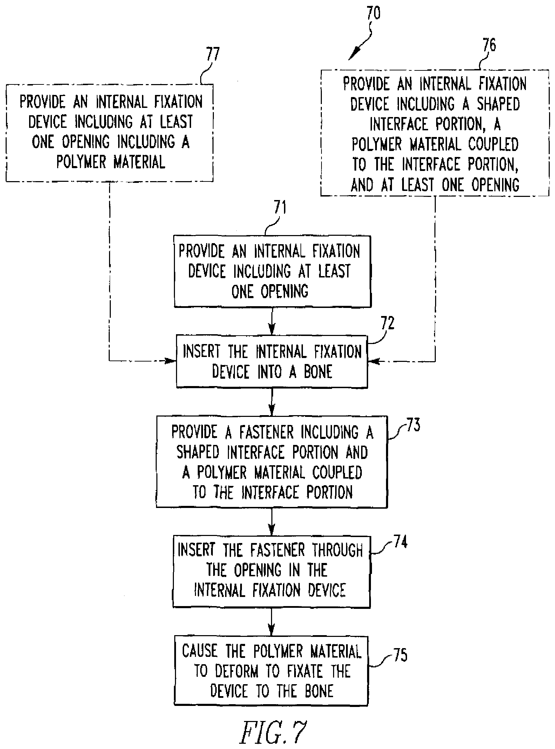

For the purposes of this disclosure, the device may be inserted into the bone by creating an entry point at one end of the bone **(****Figs. 6A-6B****,** **7****)** and then forcing the device through the intramedullary canal of the bone. Other methods known to one of ordinary skill in the art may also be used. Also for the purposes of this disclosure, the polymer material is processed to have shape memory qualities and therefore changes shape or deforms by shrinking axially, or along the length of the material, and expanding radially, or along the width of the material. Although, in certain instances, it is possible for the material to shrink radially and expand axially. This expansion and shrinkage causes an interference fit between the polymer material and the bone, thereby fixating the internal fixation device to the bone.

Generally, polymers that display shape memory qualities show a large change in modulus of elasticity at the glass transition temperature (T_{g}). The shape-memory function can be achieved by taking advantage of this characteristic. Namely, a molded article (primary molded article) to which a definite shape (the original shape) has been imparted by a common method for molding plastics, is softened by providing the article with energy and heating to a temperature (T_{f}) higher than the T_{g} of the polymer, but lower than the melting temperature (Tₘ) thereof so as to deform it into a different shape. Next, the molded article is cooled to a temperature lower than the T_{g}, while maintaining the thus deformed shape (secondary molded article). When it is heated again to a temperature higher than the secondary molding temperature T_{f}, but lower than the Tₘ, the shape of the secondary molded article disappears and thus the article is recovered to the original shape of the primary molded article.

For the purposes of this disclosure, a molded article (i.e. the above-mentioned sleeve or strips), having a definite shape (original shape) is formed from polymer material and is provided with energy to heat the article to a temperature above the glass transition temperature of the polymer, but lower than the melting temperature (Tₘ) thereof so as to deform it into a different shape and effectively wedge the article between the fixation device and the bone. In this manner, the fixation device becomes fixed to the bone. However, rather than cooling the article and heating it again until it recovers its original shape, the article is kept in this deformed shape so as to maintain fixation of the device to the bone. The glass transition temperature of the polymer material will vary based on a variety of factors, such as molecular weight, composition, structure of the polymer, and other factors known to one of ordinary skill in the art. Examples of adding energy to heat the polymer material are described below.

Examples of the method in Fig. 5 are shown in Figs. 6A and 6B. The polymer material **61** is provided with thermal energy, or heat, upon deliverance of a liquid **62,** such as saline, either through the internal fixation device **63,** as shown in Fig. 6A, or around the internal fixation device **63,** as shown in Fig. 6B. The liquid **62** is delivered via a syringe **64** or other method of delivery known to one of ordinary skill in the art. The liquid **62,** which may be something other than saline, has a high enough temperature so that the heat transferred from the liquid **62** to the polymer material **61** will take the temperature of the polymer material **61** above its glass transition temperature. As mentioned above, once the material **61** reaches a temperature that is above its glass transition temperature, the material **61** expands radially **68,** or along the width of the material **61,** and shrinks axially **69,** or along the length of the material **61.** The volume of the liquid 62 delivered is such that it has the capacity to include the thermal energy necessary to take the temperature of the material 61 above its glass transition temperature. The volume of the liquid 62 may also be dependent on the volume of the material 61 that is used.

It is also within the scope of this disclosure that once the device **63** is placed in the bone, body heat would be transferred from blood and tissue, via thermal conduction, to provide the energy necessary to deform the polymer material **61.** In this instance, body temperature would be used as the thermal energy source.

As mentioned above, radial expansion and axial shrinkage of the polymer material **61** causes an interference fit between the polymer material **61** and the inner walls **66** of the canal portion of the bone **65** and consequently allows fixation of the internal fixation device **63** to the bone **65.** In some applications, the expansion of the material 61 extends beyond the inner wall 66 and into the cancellous bone. In still other applications, the polymer material **61** replaces the need for other fixators, such as a screw, to provide fixation. This would eliminate the difficulty and time involved with the use of guides and/or x-ray machines to detect the location of the screw holes on the fixation device after the device is placed in the bone. In addition, this would also eliminate bone and tissue necrosis that can occur during the placement of the screws after the screw holes have been located on the device. Furthermore, the possibility of the screws serving as an irritant to surrounding tissue and the need, as a result of the irritation, to perform a second operation to remove these screws, would also be eliminated. When the material **61** is used on the distal end of the device **63,** such as shown in Fig. 6A, it can be used to dynamize the device, as will be further described below. Dynamization is currently achieved by the distal screws of the device being removed several weeks or months after surgery to help encourage bone regeneration. However, with the devices of the present disclosure, the material does not need to be removed since it can slowly degrade away, thereby losing fixation distally with the bone and providing the dynamization that is desired. Also as mentioned above, the internal fixation device **63** may include multiple shaped interface portions. This would create more contact between the device **63** and the bone **65** and allow the two to share the amount of load that is placed on the bone **63.**

Another method **70** of fixating an internal fixation device to a bone is shown in Fig. 7. The method includes providing an internal fixation device that includes at least one opening extending transversely through the internal fixation device **71.** The internal fixation device is then inserted into a bone **72.** A fastener, which includes a shaped interface portion and a polymer material coupled to the interface portion, is provided **73** and inserted through the opening and the bone **74.** The polymer material is then deformed to fixate the internal fixation device to the bone **75.**

The method may further include providing an internal fixation device having a shaped interface portion and a polymer material coupled to the interface portion **76.** The polymer material may then be deformed to fixate the internal fixation device to the bone. In addition, the method may also include the opening having a polymer material **77.** The polymer material may then be heated to expand the polymer material radially inward and fixate the fastener in the opening.

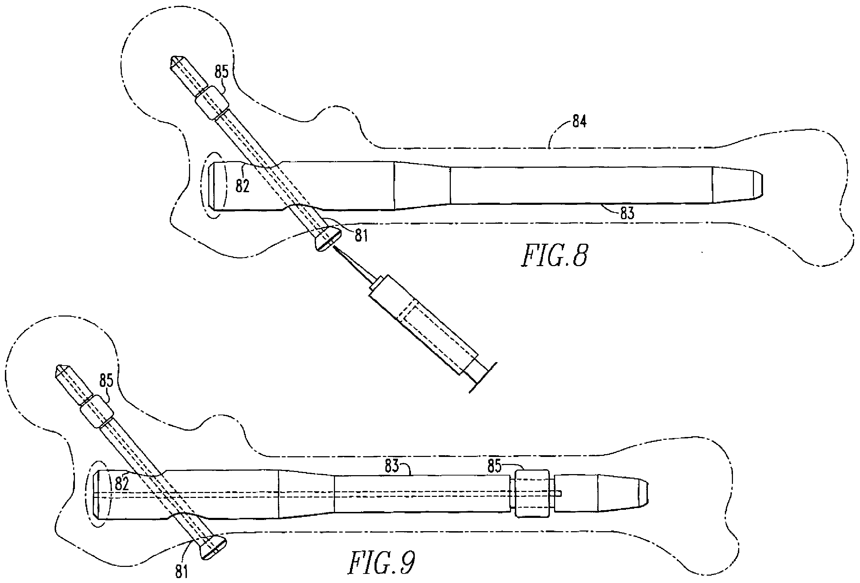

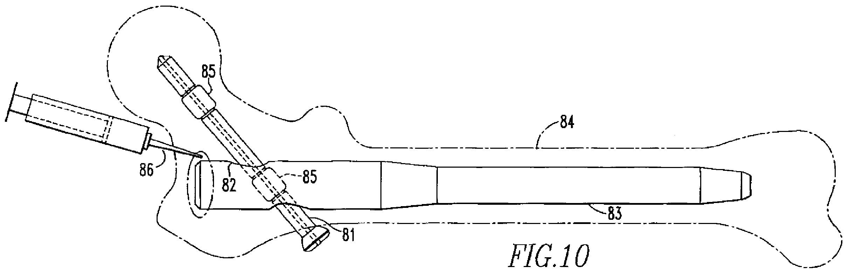

Examples of this method are shown in Figs. 8-10. In Fig. 8, the fastener **81** is located in the opening **82** of the internal fixation device **83,** which is an intramedullary nail. The fastener **81** extends through the opening **82** and the bone **84.** The opening **82** can be located anywhere along the intramedullary nail **83** and more than one opening may be present on the nail **83.** The polymer material **85** on the screw **81** is deformed via any of the methods as described above. In Fig. 9, both the intramedullary nail **83** and the fastener **81** make use of a polymer material **85** to fixate the intramedullary nail **83.** In Fig. 10, the polymer material **85** is located in both the opening **82** of the intramedullary nail **83** and on the fastener **81** and is deformed after insertion of the fastener **81** into the opening **82.** Deforming the polymer material **85** aids in fixating the intramedullary nail **83** by fixating the fastener **81** in the opening **82.** In other embodiments, the polymer material **85** may only be located in the opening **82,** rather than in the opening **82** and on the fastener **81.** When the material **85** is located in the opening **82,** it is coupled to the inner walls of the opening and radial expansion of the material **85** occurs inwardly towards the fastener **81** when the material **85** is provided with energy. An example of a fastener includes a screw, pin, rod, or any other device used to fixate the intramedullary nail in the bone.

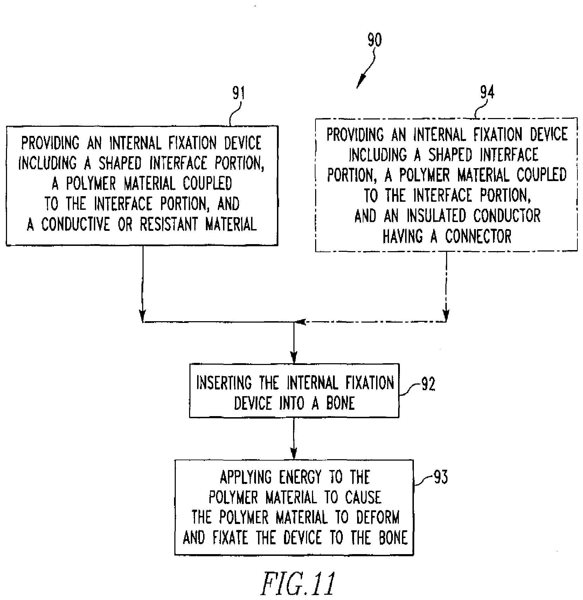

A further method **90** of fixating an internal fixation device to a bone is shown in Fig. 11. The method includes providing an internal fixation device that includes a shaped interface portion and a polymer material coupled to the interface portion, wherein the internal fixation device includes a conductive material **91.** The internal fixation device is then inserted into a bone **92** and energy is applied to the conductive material **93.** The energy is transferred from the conductive material to the polymer material and the polymer material expands radially and shrinks axially to fixate the internal fixation device to the bone. The internal fixation device may have an insulated conductor that includes a connector **94.** The connector is able to receive an electrical source that provides heat to the insulated conductor via an electrical current. The heat is transferred from the insulated conductor to the polymer material and the polymer material expands to create an interference fit between the bone and the internal fixation device and allow the device to better engage the bone.

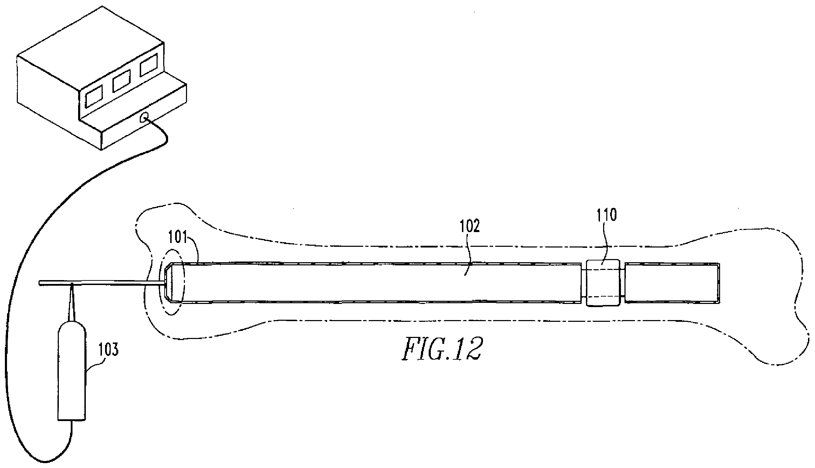

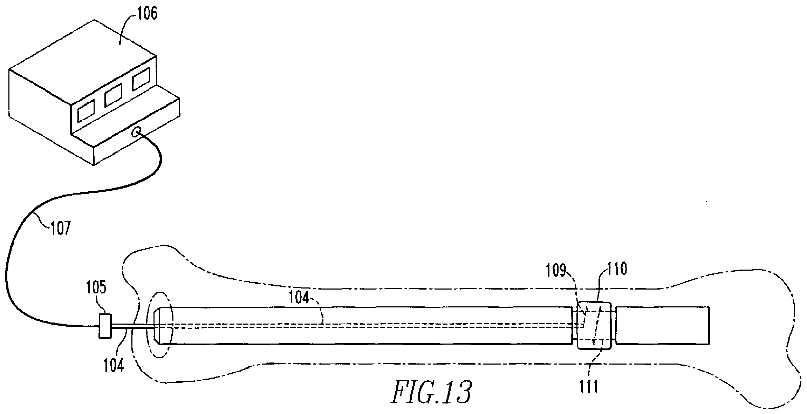

Examples of this method are shown in Figs. 12 and 13. In Fig. 12, thermal energy, or heat, is applied to the conductive material **101** of the intramedullary nail **102** via a heat generating device **103,** namely a cauterizing device. The heat is transferred from the conductive material, via thermal conduction, to the polymer material **110,** causing the polymer material **110** to deform. The conductive material **101** may be in the form of a sheath or sleeve that is placed over the device **102** or portions thereof, strips that are coupled to the device **102,** or another form. In Fig. 13, the end of the insulated conductor **104** contains a connector **105** to allow electrical sources to connect to it and provide it with electrical energy, or an electrical current. Alternatively, the connector 105 may be coupled to another connector located at the end of the nail 102. The electrical energy extends the length of the insulated conductor **104** to the shaped interface portion **111.** At the shaped interface portion **111,** the conductor **104** is non-insulated, or exposed, and comes into contact with heating elements **109.** The heat from these elements **109** causes the polymer material **110** to deform. The heating elements **109** shown in Fig. 13 are coils, but may be any other type of heating element known to one of ordinary skill in the art. The device that provides the current is a hand held battery powered device **106** which connects to the connector **105** via wires **107.** The button **108** on the device **106** need only be activated once and the appropriate current is delivered. Other devices known to those of ordinary skill in the art for providing current may be used, such as, but not limited to, an electrosurgical generator. In addition, other heat generating devices known to those of ordinary skill in the art may be used, such as, but not limited to, a hot air gun, a small welding or soldering gun, ultrasonic welders, a bovie tip, infrared light, or lasers.

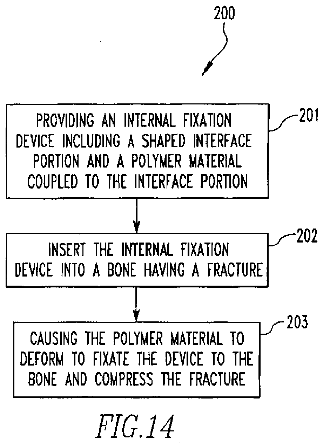

Fig. 14 shows a method of compressing a fractured bone **200.** The method includes providing an internal fixation device that includes a shaped interface portion and a polymer material coupled to the interface portion **201.** The internal fixation device is then inserted into a bone having a fracture **202** and the polymer material is provided with energy to deform the material. Deforming the polymer material fixates the internal fixation device to the bone and cause compression of the fracture **203.**

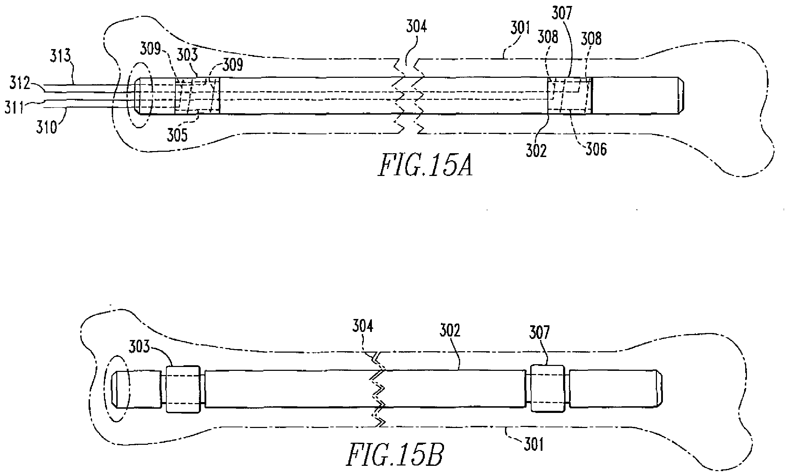

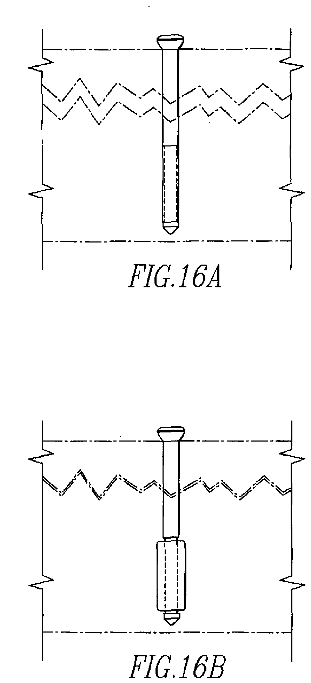

Examples of this method are shown in Figs. 15A-B and 16A-B. Fig. 15A shows a fractured bone **301** having an intramedullary nail **302** inserted through the bone **301.** Fig. 15B shows the fractured bone **301** after the polymer material **303** has been provided with heat. It can be seen that by providing the polymer material **303** with heat, deformation of the material **303** occurs creating not only an interference fit between the intramedullary nail **302** and bone **301,** but also compression of the fracture **304.** As shown in Fig. 15A, there are a pair of heating elements or coils **308,309** located at both of the shaped interface portions **305,306** with two conductors **311,312** connected to coils **308** and two conductors **310,313** connected to coils **309.** Electrical energy is fed through the insulated conductors **310,312** to the heating elements **308,309** that are furthest away from the fracture **304** and heat is applied to the polymer material **303,307.** Once the polymer material **303,307** in the area of these elements **308,309** begins to deform, electrical energy is fed through the insulated conductors **311,313** to the heating elements **308,309** that are closest to the fracture **304,** heat is applied to the polymer material **303,307** in the area of these elements **308,309,** and the polymer material **303,307** deforms. Upon deforming, the material **303,307** expands radially to fixate the device **302** to the bone **301** and shrinks axially to compress the fracture **304.**

Compression is achieved by applying heat to the polymer material **303,307** in a non-uniform manner, so as to control the direction that the axial shrinking is occurring. A fifth conductor (not shown) would be used as the ground for the coil circuits. Figs. 16A-B show similar examples of fracture compression with a bone screw. Compression by the bone screw could occur in a manner similar to the nail **302** in Figs. 15A and 15B. Any heating element known to one of ordinary skill in the art could be used. Also, any number of heating elements and conductors may be used together to deform the material. In addition, the conductors may be located on the inner wall of the internal fixation device, on the outer wall of the internal fixation device, or in the body of the internal fixation device. Furthermore, compression of the fracture could occur by another method known to one of ordinary skill in the art.

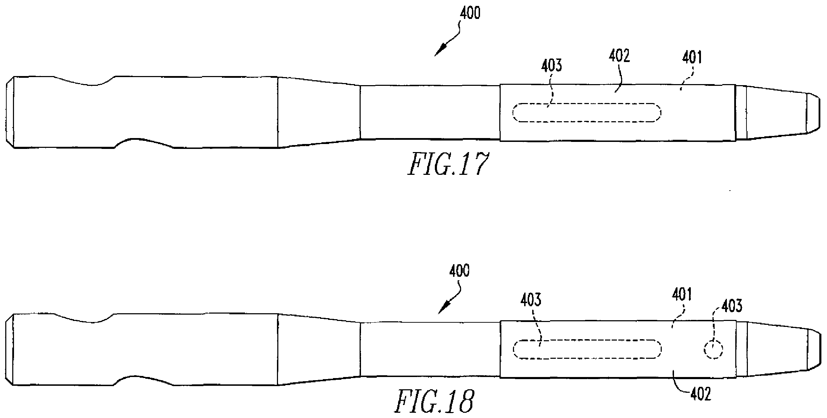

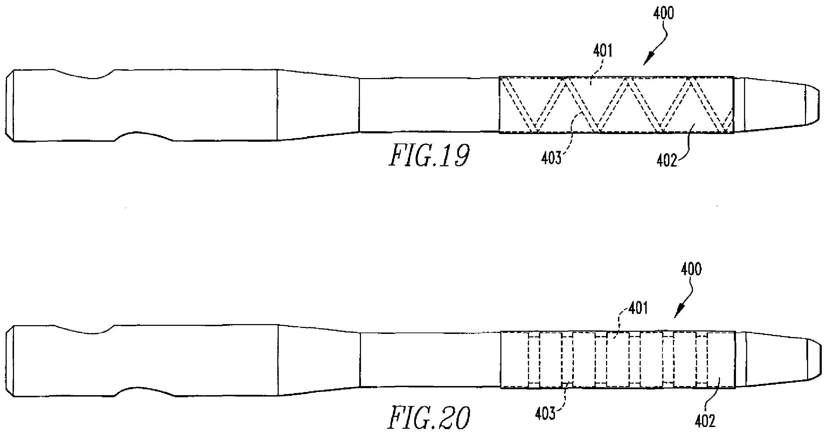

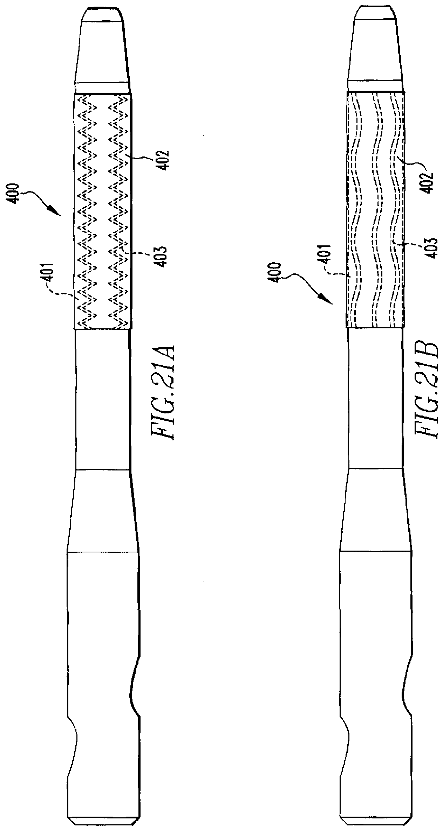

Figs. 17 and 18 show internal fixation devices **400** that include an interface portion **401** and a polymer material **402** coupled to the interface portion **401.** The internal fixation devices **400** are intramedullary nails, but could be any other internal fixation device that is used in the repair of bone fractures, such as a bone screw, a locking screw, a rod, or a pin. The internal fixation devices **400** include at least one hole **403** and, as shown in Fig. 18, may include multiple holes **403** at the interface portion. Fig. 19 shows another internal fixation device **400** having an interface portion **401** that includes threads **403.** Fig. 20 shows yet another internal fixation device **400** having an interface portion **401** that includes circumferential ribs **403.** Figs. 21A and 21B show internal fixation devices **400** having interface portions **401** that include engravings **403.** All of the devices **400** disclosed in Figs. 17-21 include a polymer material **402** coupled to the interface portion **401.** In addition to allowing formation of bonds between the polymer material **402** and the interface portion **401** once the polymer material **402** is provided with energy, these holes, threads, circumferential ribs, and engravings **403** help the polymer material **402** engage the device **400** to provide support for axial and torsional loading and to substantially reduce motion in those directions after the device **400** has been placed in the bone, as will be further described below. The holes, threads, circumferential ribs, and engravings **403** can be drilled, machined, molded, cast, laser cut, or chemically etched into the internal fixation device or formed via another method known to one of ordinary skill in the art. Machining could take many forms, including wire and ram electrical discharge machining (EDM). The interface portion **401** may be located anywhere along the device **400.**

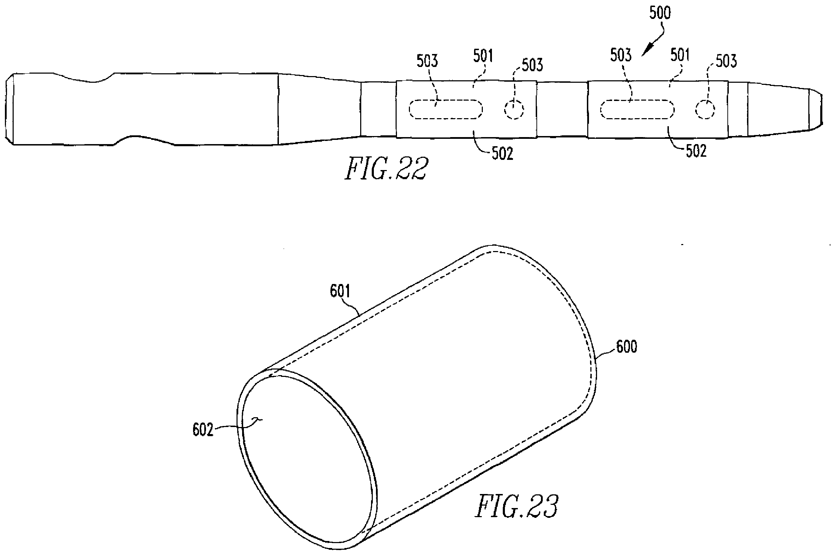

In addition, as shown in Fig. 22, multiple interface portions **501,** including a polymer material **502,** may be present on the internal fixation device **500.** The portions **501** include holes **503,** but may include the above-shown threads, circumferential ribs, engravings, or combinations thereof. In addition, the interface portions **501** may be present anywhere along the internal fixation device **500.**

The polymer material could include a sleeve of material having a uniform structure with an outside surface and a channel running through the middle of the structure with both the structure and the channel having the same or different shapes. For the purposes of Figs. 17-22, and as shown in Fig. 23, the polymer material is in the form of a sleeve **600** having a cylindrical structure with an outside surface **601** that is circular and a channel **602** having a circular shape to match the circular shape of the interface portion. However, the structure of the sleeve **600** and the channel **602** may have another shape. The sleeve **600** may be formed by die-drawing or molding (i.e. compression flow molding or thermoforming process) the above-mentioned polymers or polymer compositions. The channel **602** may be formed in the sleeve **600** during the die drawing or molding process. Alternatively, the channel **602** may be formed in the sleeve **600** post processing by drilling or by any other method of forming the channel **602.**

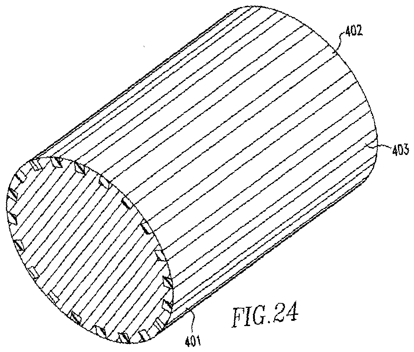

In addition, the polymer material may not be in the form of sleeve, but rather there may be several strips of polymer material each of which have a structure and each of which are coupled to the interface portion. For example, Fig. 24 shows an interface portion **401** having strips of polymer material **402** coupled to the engraved areas **403** of the interface portion **401.** The strips of polymer material **402** may be formed to fit the design of the engraving **403** or may be in other forms. The strips **402** may be formed by the processes listed above or by another process, such as an extrusion process (i.e. single screw, twin screw, disk, ram, or pulltrusion process).

Furthermore, for the purposes of this disclosure, the outer surface of the polymer material is shown, in Figs. 17-22, as being flush, or forming the same plane with, the outer surface of the fixation device. However, the outer surface of the polymer material may be of a larger diameter than the outer surface of the fixation device.

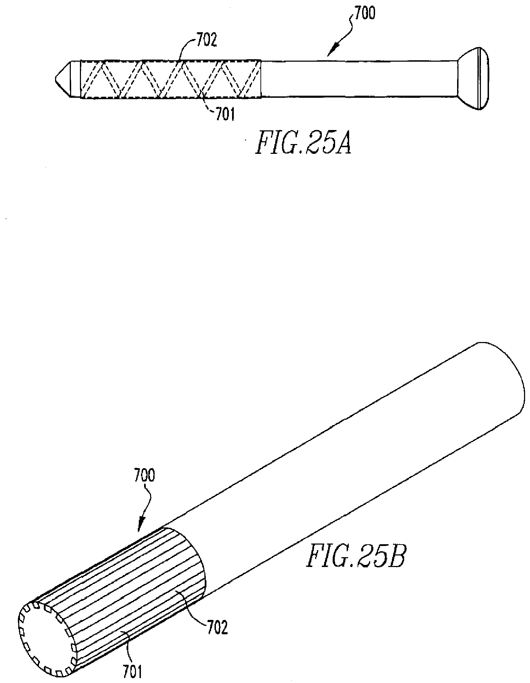

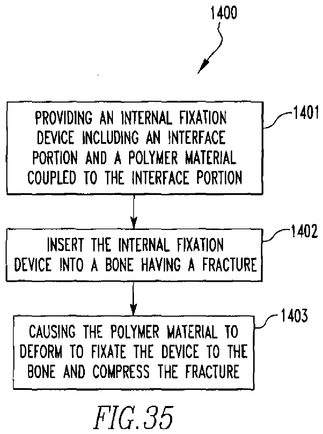

Figs. 25A and 25B show further examples of an internal fixation device **700** that includes an interface portion **701** and a polymer material **702** coupled to the interface portion **701.** The internal fixation devices **700** of Fig. 25A and 35B include a screw, which could be a bone screw or locking screw, and a rod, respectively. The physical and compositional properties of the intramedullary nail, interface portion, and polymer material, as described above, also apply to the internal fixation devices shown in Figs. 25A and 25B.

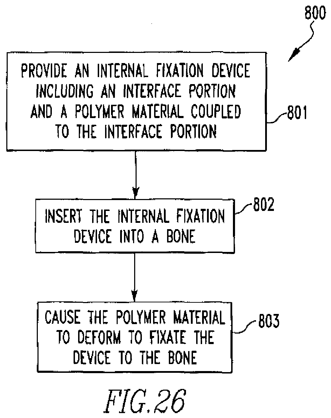

Fig. 26 shows a first method of fixating an internal fixation device to a bone **800.** An internal fixation device is provided that includes an interface portion and a polymer material coupled to the interface portion **801.** The internal fixation device is then inserted into a bone **802** and the polymer material is caused to deform **803,** thereby fixating the internal fixation device to the bone.

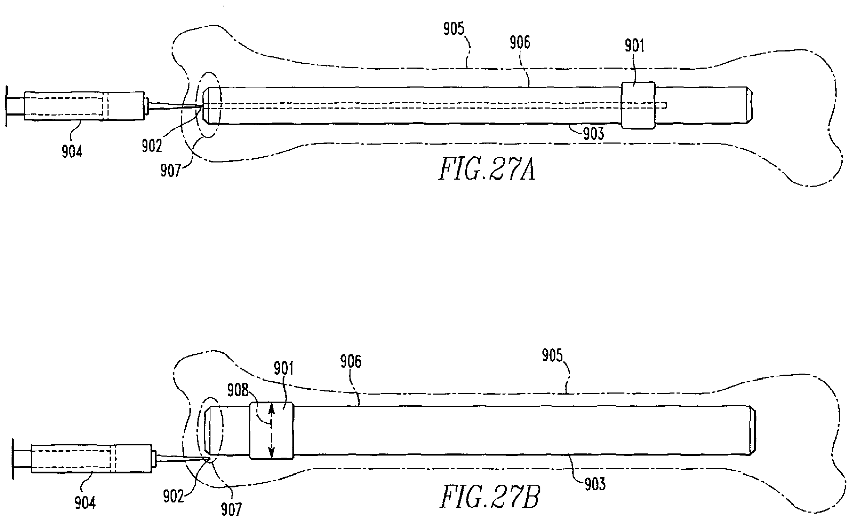

For the purposes of this disclosure, the device may be inserted into the bone by creating an entry point at one end of the bone **(****Figs. 27A-27B****, 907)** and then forcing the device through the intramedullary canal of the bone. Depending on the type of device that is inserted, other methods known to one of ordinary skill in the art may also be used. For example, the device may be entered at another point on the bone.

Examples of the method in Fig. 26 are shown in Figs. 27A and 27B. The polymer material **901** is provided with thermal energy, or heat, upon deliverance of a liquid **902,** such as saline, either through the internal fixation device **903,** as shown in Fig. 27A, or around the internal fixation device **903,** as shown in Fig. 27B. The liquid **902** is delivered via a syringe **904** or other method of delivery known to one of ordinary skill in the art. The liquid **902,** which may be something other than saline, has a high enough temperature so that the heat transferred from the liquid **902** to the polymer material **901** will take the temperature of the polymer material **901** above its glass transition temperature. As mentioned above, once the material **901** reaches a temperature that is above its glass transition temperature, the material **901** expands radially, or along the width of the material **901,** and shrinks axially, or along the length of the material **901.** The volume of liquid 902 delivered is such that it has the capacity to include the thermal energy necessary to take the temperature of the material 901 above its glass transition temperature. The volume of the liquid 902 may also be dependent on the volume of the material 901 that is used.

It is also within the scope of this disclosure that once the device **903** is placed in the bone, body heat would be transferred from blood and tissue, via thermal conduction, to provide the energy necessary to deform the polymer material **901.** In this instance, body temperature would be used as the thermal energy source.

As mentioned above, radial expansion and axial shrinkage of the polymer material **901** causes an interference fit between the polymer material **901** and the inner walls **906** of the canal portion of the bone **905** and consequently allows fixation of the internal fixation device **903** to the bone **905.** In some applications, the expansion of the material 901 extends beyond the inner wall 906 and into the cancellous bone. In some applications, the polymer material **901** replaces the need for other fixators, such as a screw, to provide fixation. This would eliminate the difficulty and time involved with the use of guides and/or x-ray machines to detect the location of the screw holes on the fixation device after the device is placed in the bone. In addition, this would also eliminate bone and tissue necrosis that can occur during the placement of the screws after the screw holes have been located on the device. Furthermore, the possibility of the screws serving as an irritant to surrounding tissue and the need, as a result of the irritation, to perform a second operation to remove these screws, would also be eliminated. When the material **901** is used on the distal end of the device **903,** such as shown in Fig. 27A, it can be used to dynamize the device, as will be further described below. Dynamization is currently achieved by the distal screws of the device being removed several weeks or months after surgery to help encourage bone regeneration. However, with the devices of the present disclosure, the material does not need to be removed since it can slowly degrade away, thereby losing fixation distally with the bone and providing the dynamization that is desired. Also as mentioned above, the internal fixation device **903** may include multiple shaped interface portions. This would create more contact between the device **903** and the bone **905** and allow the two to share the amount of load that is placed on the bone **903.**

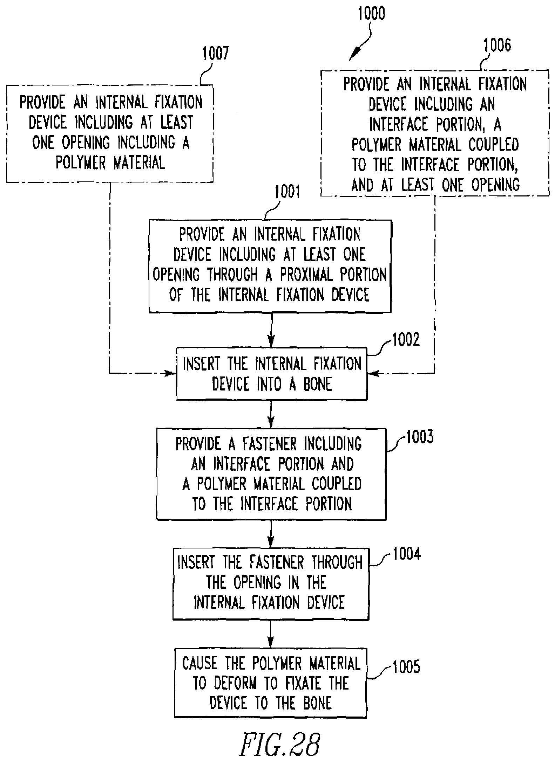

Another method **1000** of fixating an internal fixation device to a bone is shown in Fig. 28. The method includes providing an internal fixation device that includes at least one opening extending transversely through a proximal portion of the internal fixation device **1001.** The internal fixation device is then inserted into a bone **1002.** A fastener, which includes an interface portion and a polymer material coupled to the interface portion, is provided **1003** and inserted through the opening and the bone **1004.** The polymer material is then deformed to fixate the internal fixation device to the bone **1005.**

The method may further include providing an internal fixation device having an interface portion and a polymer material coupled to the interface portion **1006.** The polymer material may then be deformed to fixate the internal fixation device to the bone. In addition, the method may also include the opening having a polymer material **1007.** The polymer material may then be heated to expand the polymer material radially inward and fixate the fastener in the opening.

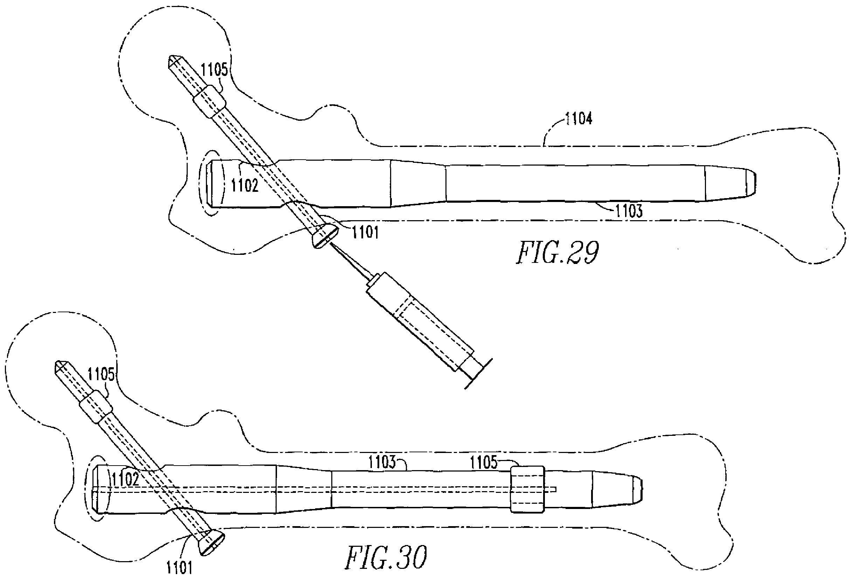

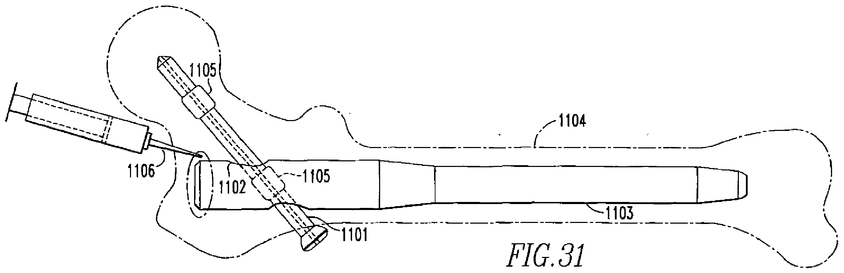

Examples of this method are shown in Figs. 29-31. In Fig. 29, the fastener **1101** is located in the opening **1102** of the internal fixation device **1103,** which is an intramedullary nail. The fastener **1101** extends through the opening **1102** and the bone **1104.** The opening **1102** can be located anywhere along the intramedullary nail **1103** and more than one opening may be present on the nail **1103.** The polymer material **1105** on the screw **1101** is deformed via any of the methods as described above. In Fig. 30, both the intramedullary nail **1103** and the fastener **1101** make use of a polymer material **1105** to fixate the intramedullary nail **1103.** In Fig. 31, the polymer material **1105** is located in both the opening **1102** of the intramedullary nail **1103** and on the fastener **1101** and is deformed after insertion of the fastener **1101** into the opening **1102.** Deforming the polymer material **1105** aids in fixating the intramedullary nail **1103** by fixating the fastener **1101** in the opening **1102.** In other embodiments, the polymer material **1105** may only be located in the opening **1102,** rather than in the opening **1102** and on the fastener **1101.** When the material **1105** is located in the opening **1102,** it is coupled to the inner walls of the opening and radial expansion of the material **1105** occurs inwardly towards the fastener **1101** when the material **1105** is provided with energy. An example of a fastener includes a screw, pin, rod, or any other device used to fixate the intramedullary nail in the bone.

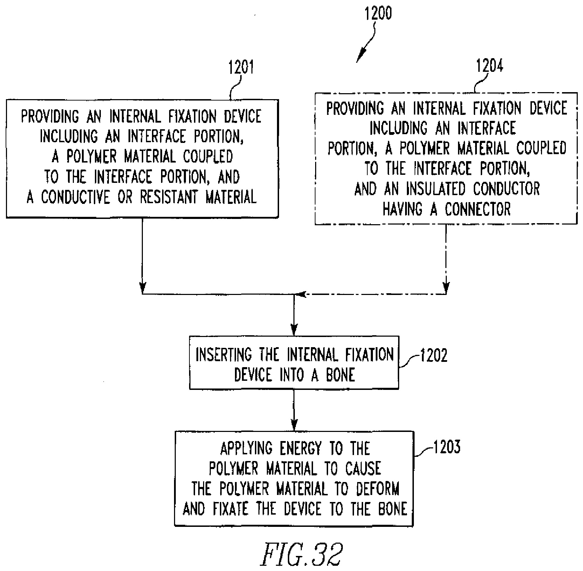

A further method **1200** of fixating an internal fixation device to a bone is shown in Fig. 32. The method includes providing an internal fixation device that includes an interface portion and a polymer material coupled to the interface portion, wherein the internal fixation device includes a conductive material **1201.** The internal fixation device is then inserted into a bone **1202** and energy is applied to the conductive material **1203.** The energy is transferred from the conductive material to the polymer material and the polymer material expands radially and shrinks axially to fixate the internal fixation device to the bone. The internal fixation device may have an insulated conductor that includes a connector **1204.** The connector is able to receive an electrical source that provides heat to the insulated conductor via an electrical current. The heat is transferred from the insulated conductor to the polymer material and the polymer material expands to create an interference fit between the bone and the internal fixation device and allow the device to better engage the bone.

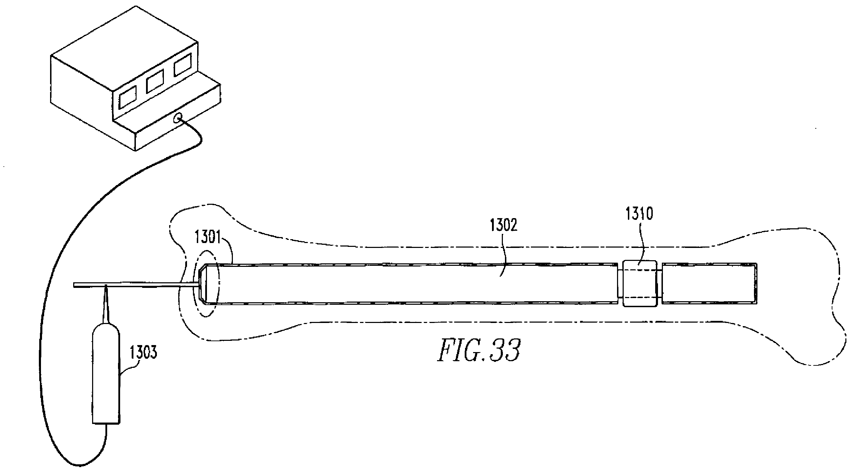

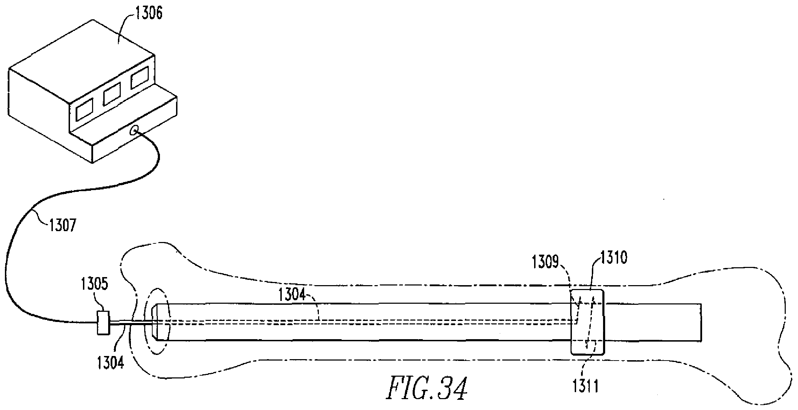

Examples of this method are shown in Figs. 33 and 34. In Fig. 33, thermal energy, or heat, is applied to the conductive material **1301** of the intramedullary nail **1302** via a heat generating device **1303,** namely a cauterizing device. The heat is transferred from the conductive material, via thermal conduction, to the polymer material **1310,** causing the polymer material **1310** to deform. The conductive material **1301** may be in the form of a sheath or sleeve that is placed over the device **1302** or portions thereof, strips that are coupled to the device **1302,** or another form. In Fig. 34, the end of the insulated conductor **1304** contains a connector **1305** to allow electrical sources to connect to it and provide it with electrical energy, or an electrical current. The electrical energy extends the length of the insulated conductor **1304** to the shaped interface portion **1311.** At the interface portion **1311,** the conductor **1304** is non-insulated, or exposed, and comes into contact with heating elements **1309.** The heat from these elements **1309** causes the polymer material **1310** to deform. The heating elements **1309** shown in Fig. 34 are coils, but may be any other type of heating element known to one of ordinary skill in the art. The device that provides the current is a hand held battery powered device **1306** which connects to the connector **1305** via wires **1307.** The button **1308** on the device **1306** need only be activated once and the appropriate current is delivered. Other devices known to those of ordinary skill in the art for providing current may be used, such as, but not limited to, an electrosurgical generator. In addition, other heat generating devices known to those of ordinary skill in the art may be used, such as, but not limited to, a hot air gun, a small welding or soldering gun, ultrasonic welders, a bovie tip, infrared light, or lasers.

Fig. 35 shows a method of compressing a fractured bone **1400.** The method includes providing an internal fixation device that includes an interface portion and a polymer material coupled to the interface portion **1401.** The internal fixation device is then inserted into a bone having a fracture **1402** and the polymer material is provided with energy to deform the material. Deforming the polymer material fixates the internal fixation device to the bone and cause compression of the fracture **1403.**

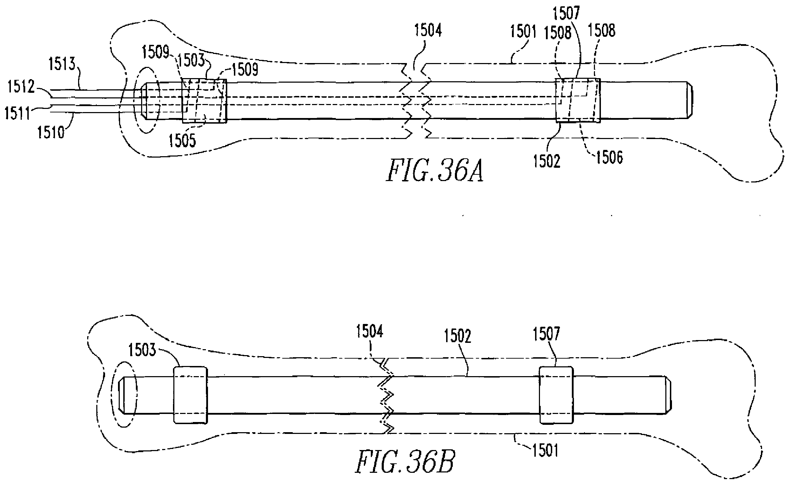

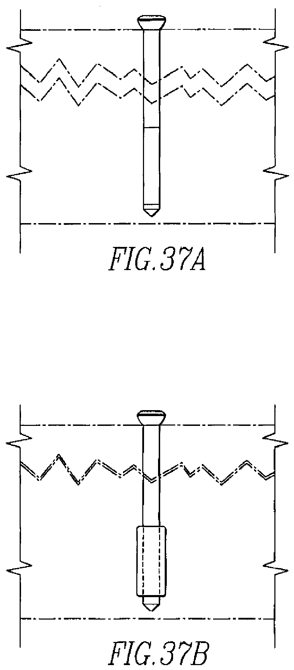

Examples of this method are shown in Figs. 36A-B and 37A-B. Fig. 36A shows a fractured bone **1501** having an intramedullary nail **1502** inserted through the bone **1501.** Fig. 36B shows the fractured bone **1501** after the polymer material **1503** has been provided with heat. It can be seen that by providing the polymer material **1503** with heat, deformation of the material **1503** occurs creating not only an interference fit between the intramedullary nail **1502** and bone **1501,** but also compression of the fracture **1504.** As shown in Fig. 36A, there is a pair of heating elements or coils **1508,1509** located at both of the interface portions **1505,1506** with two conductors **1511,1512** connected to coils **1508** and two conductors **1510,1513** connected to coils **1509.** Electrical energy is fed through the insulated conductors **1510,1512** to the heating elements **1508,1509** that are furthest away from the fracture **1504** and heat is applied to the polymer material **1503,1507.** Once the polymer material **1503,1507** in the area of these elements **1508,1509** begins to deform, electrical energy is fed through the insulated conductors **1511,1513** to the heating elements **1508,1509** that are closest to the fracture **1504,** heat is applied to the polymer material **1503,1507** in the area of these elements **1508,1509,** and the polymer material **1503,1507** deforms. Upon deforming, the material **1503,1507** expands radially to fixate the device **1502** to the bone **1501** and shrinks axially to compress the fracture **1504.**

Compression is achieved by applying heat to the polymer material **1503,1507** in a non-uniform manner, so as to control the direction that the axial shrinking is occurring. A fifth conductor (not shown) would be used as the ground for the coil circuits. Figs. 37A-B show similar examples of fracture compression with a bone screw. Compression by the bone screw could occur in a manner similar to the nail **1502** in Figs. 36A and 36B. Any heating element known to one of ordinary skill in the art could be used. Also, any number of heating elements and conductors may be used together to deform the material. In addition, the conductors may be located on the inner wall of the internal fixation device, on the outer wall of the internal fixation device, or in the body of the internal fixation device. Furthermore, compression of the fracture could occur by another method known to one of ordinary skill in the art.

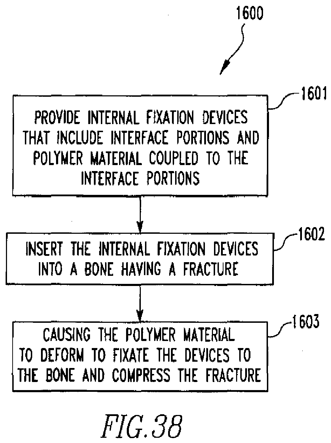

Fig. 38 shows another method of compressing a bone fracture **1600.** The method **1600** includes providing internal fixation devices that have interface portions and a polymer material coupled to the interface portions **1601.** The internal fixation devices are then inserted into a bone having a fracture **1602** and the polymer material is provided with energy, by one of the methods mentioned above, or another method known to one of ordinary skill in the art, to deform the material. Deforming the polymer material fixates the internal fixation devices to the bone and causes compression of the fracture **1603.**

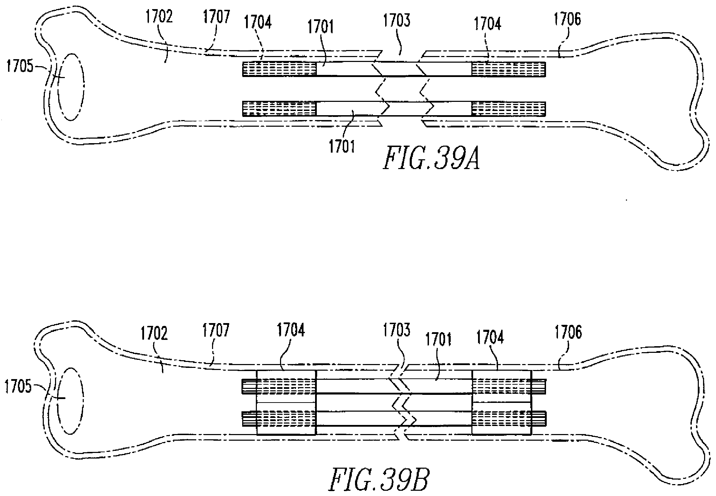

An example of this method is shown in Figs. 39A and 39B. The internal fixation devices **1701,** shown as rods, are inserted into the intramedullary canal, through the entry point **1705** at the one end of the bone **1702,** and are placed across the fracture site **1703** until the canal is full. The polymer material **1704** is then deformed, by one of the methods mentioned above, or another method known to one of ordinary skill in the art. During deformation, the material **1704** expands vertically such that contact is made with the endosteal surface **1706** of the cortical wall **1707** and with the polymer material **1704** that is located on the other devices **1701** within the canal. In addition to expanding vertically, during deformation the material **1704** also shrinks horizontally. This simultaneous expansion and shrinkage of the material **1704** respectively fixates the device **1701** to the bone **1702** and compresses the fracture **1703,** as shown in Fig. 39B. In some applications, the material 1704 expands vertically beyond the endosteal surface and into the cancellous bone. The interface portion and polymer material may be located anywhere along the devices. In addition, instead of having an interface portion to which the polymer material is coupled, the devices may include both metal material and polymer material located in alternating sections along the body of the device. It is also possible for the devices to be made completely out of polymer material that is resorbable and includes shape memory qualities, doesn't include shape memory qualities, or have a combination of both. Furthermore, other alternative embodiments are also within the scope of this disclosure. For example, a mixture of devices that include polymer material having shape memory qualities and devices that include polymer material that does not have shape memory qualities could be used. Also, the fracture may be stabilized by inserting resorbable polymeric rods into the intramedullary canal and filling the remaining space with an injectable, in-situ cured biodegradable thermoset matrix. Providing the canal with the thermoset matrix adds strength to the rod/thermoset matrix construct in bending, torsion, and shear. Any thermoset matrix known to one of ordinary skill in the art may be used. Alternatively, in order to contain the matrix and prevent the injected liquid from running out of bone at the fracture site, a bag can be pushed down the canal and filled with the rods, fibers, or particles. The bag may be made of a solid film, shape memory tubes, or of woven fibers. When the cement or thermoset matrix is injected, the liquid will flow through the canal and will wet the entire construct without spilling into soft tissue through the fracture.

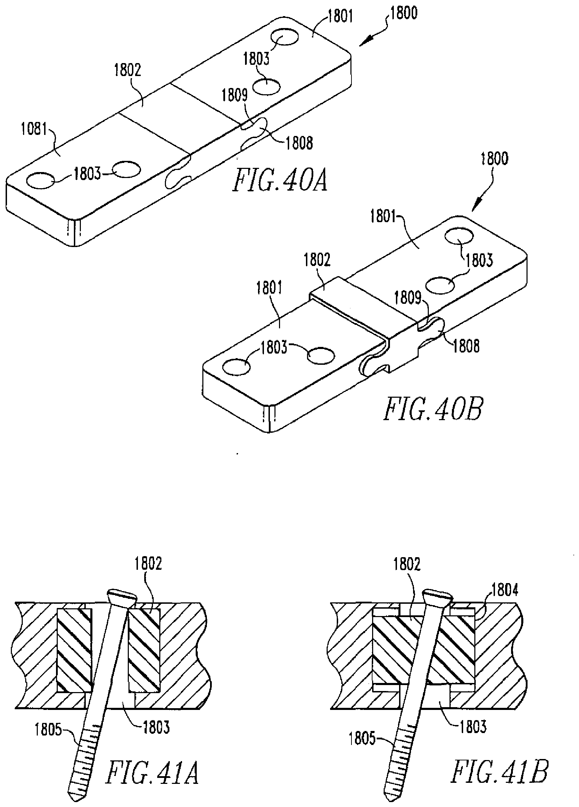

Figs. 40A and 40B show another embodiment of an internal fixation device **1800** of the present disclosure. The plate **1800** in Figs. 40A and 40B includes two end sections **1801,** both of which are constructed of a first material, and a middle section **1802** that includes a second material. For the purposes of this figure, the first material is a metal material and the second material is a polymer material having shape memory qualities. However, it is within the scope of this figure that the two end sections **1801** could include a non-metal material, a combination of metal and non-metal materials, or the sections **1801** could include different types of material with one end section **1801** including a metal material and the other end section **1801** including a non-metal material. The end sections **1801** are coupled to the middle section **1802** via an engagement whereby grooves **1809** on the end sections **1801** are shaped to interlock with tabs **1808** that are located on the middle section **1802.** However, other means known to those of ordinary skill in the art, of coupling the middle section **1802** to the end sections **1801,** may be used. The end sections **1801** include holes **1803** that extend through the device **1800.** As described further below, the device **1800** is coupled to a bone by inserting fasteners through the holes **1803** and into the bone. As shown in Fig. 40B, the polymer material **1802** deforms upon the application of energy, via one of the methods described above, or another method known to one of ordinary skill in the art. It is also within the scope of this disclosure for the plate **1800** to include multiple sections of polymer material **1802.**

Figs. 41A and 41B show that the inner walls **1804** of the holes **1803** may also include polymer material **1802.** Once a fastener **1805** is inserted into the hole **1803,** the polymer material **1802** may provided with energy, via one of the methods described above, or another method known to one of ordinary skill in the art, to deform the material **1802** and fixate the fastener **1805** within the hole **1803,** as shown in Fig. 41B. For the purposes of this disclosure, the polymer material may be used on the inner walls of holes that are located on devices other than plates.

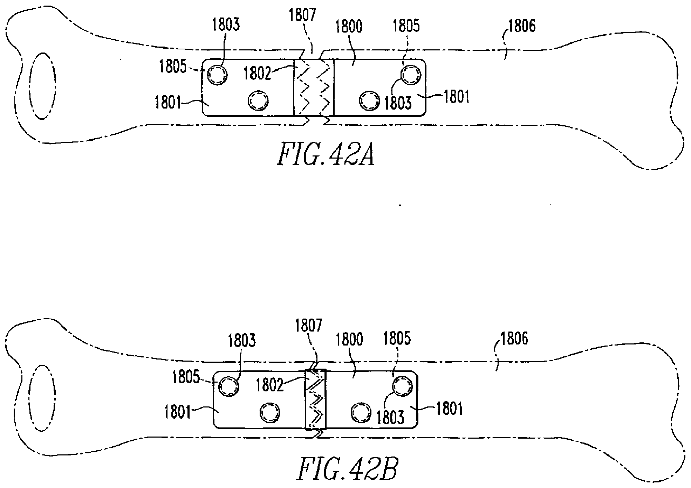

Figs. 42A and 42B show a fractured bone **1806** both before and after compression of the fracture **1807** by the plate **1800.** The plate **1800** is placed on the bone **1806** such that the middle section **1802** is located over the fracture **1807.** The plate **1800** is then coupled to the bone **1806** by inserting fasteners **1805** through the holes **1801** and into the bone **1806.** Energy is then applied to the polymer material **1802,** via one of the methods described above, or another method known to one of ordinary skill in the art, to deform the material **1802** and compress the fracture **1807,** as shown in Fig. 42B.

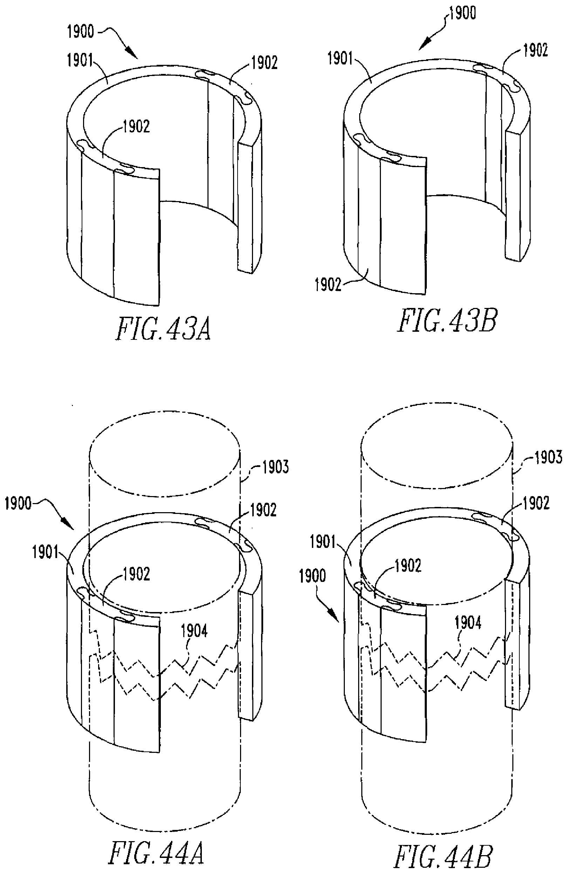

Figs. 43A and 43B show an alternative plate **1900,** in the shape of a bracelet, which includes alternating sections of material **1901,1902.** For the purposes of this figure, sections **1901** include metal material and sections **1902** include polymer material having shape memory qualities. However, it is within the scope of this figure that section **1901** could include a non-metal material or a combination of metal and non-metal materials. Similar to the plate **1800** in Figs. 40A and 40B, sections **1901** and **1902** are coupled via grooves **1905** and tabs **1906** located on sections **1901** and **1902,** respectively. Also similar to Figs. 40A and 40B, the polymer material **1902** deforms upon the application of energy via a method described above, or another method known to one of ordinary skill in the art. Figs. 44A and 44B show a fractured bone **1903** both before and after fixation of the plate **1900** to the bone **1903.** The plate **1900** is placed on the bone **1903** and energy is then applied to the polymer material **1902,** via one of the methods described above, or another method known to one of ordinary skill in the art, to deform the material **1902** and fixate the plate **1900** to the bone **1903,** specifically, to ends 1904a,1904b of the fracture 1904, as shown in Fig. 44B.

The section or sections of polymer material included in the plates, of the above figures, may be located anywhere along the body of the plates. In addition, it is also possible for the devices to be made completely out of polymer material. Furthermore, the devices may include an interface portion to which the polymer material is coupled, similar to the intramedullary nails, screws, and rods shown in the above figures.

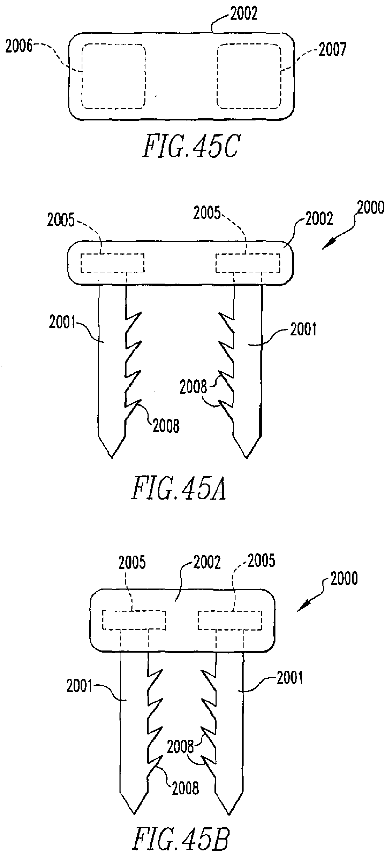

Figs. 45A-45C show another alternative fixation device in the form of a staple **2000.** The staple **2000** includes a plate **2002,** having two recesses **2006** and **2007,** and two arms **2001,** wherein each arm **2001** includes a head **2005** that is located at a proximal portion of each arm **2001.** The arms **2001** extend through the recesses **2006,2007** such that the head **2005** of each arm **2001** rests within each recess **2006,2007.** Each arm **2001** includes barbs **2008** located on an inside surface of the arm **2001** for substantially reducing the possibly of axial movement of the staple **2000** out of the bone. The number of barbs **2008** and the location of the barbs **2008** may vary. In addition, the arms may be without barbs. After the arms **2001** are inserted into each recess **2006,2007,** the top portion of each recess **2006,2007** is closed off with a piece of material that is shaped to fit the recess **2006,2007.** The arms **2001** and head **2005** include a metal material, but may include a non-metal, a combination of metal and non-metal, or the arm **2001** and the head **2005** may include different types of material. The plate **2002** includes a polymer material that includes shape-memory qualities. As shown in Fig. 45B, once energy is applied to the polymer material **2002,** the material **2002** deforms. It is also within the scope of this disclosure for polymer material to be located on one or both arms **2001** of the staple **2000,** either coupled to an interface portion of one or both of the arms or as an alternating section of material.

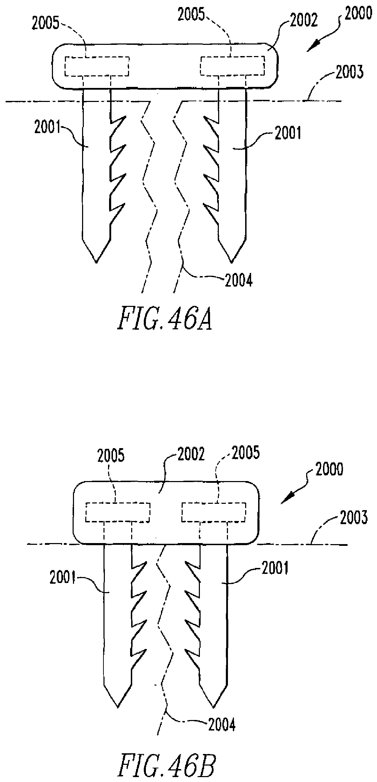

Figs. 46A and 46B show a fractured bone **2003** both before and after compression of the fracture **2004** by the staple **2000.** The staple **2000** is placed in the bone **2003** such that the legs **2001** are located on both sides of the fracture **2004.** Energy is then applied to the polymer material **2002,** via one of the methods described above, or another method known to one of ordinary skill in the art, to deform the material **2002** and compress the fracture **2004,** as shown in Fig. 46B. As stated above, polymer material may be located one or both of the arms **2001** of the staple **2000.** If polymer material is located one or both of the arms **2000,** then the polymer material may also be deformed to increase the compression on the fracture **2004** and fixation of the device **2000** to the bone **2003.**

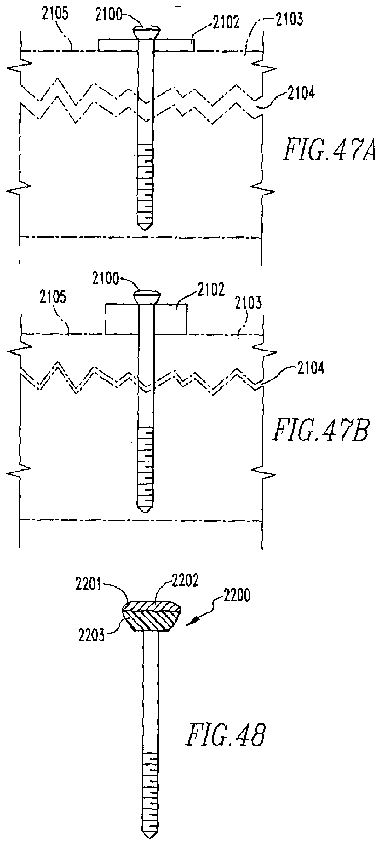

Figs. 47A and 47B show compression of a fracture **2104** via the use of a compression screw **2100** and a washer **2102** that includes polymer material having shape memory qualities. The screw **2100** is placed through the washer **2102** and across a fracture **2104.** The washer **2102** can be provided with energy immediately, via one of the methods described above, or done slowly through heat transfer from surrounding tissue and blood. This results in deformation of the washer **2102,** which pushes the head of the screw **2100** away from the surface **2105** of the bone **2103,** and causes the application of a compressive force across the fracture **2104.** The compression screw **2100** may include a polymer material

In an alternative embodiment, and as shown in Fig. 48, use of the washer may be eliminated with a screw **2200** having a head **2201** with an upper portion **2202** made from metal material and a lower portion **2203** made from polymer material. Similar to the washer in Figs. 47A and 47B, the lower portion **2203** of the head **2201** would deform upon the application of energy, via one of the methods described above, or another method known to one of ordinary skill in the art, and causes the application of a compressive force across a fracture. In some applications, the upper portion 2202 may be made from metal material.

In addition to the washer **2102** and the head **2201** of the screw **2100,2200** including a polymer material, is also within the scope of this disclosure that the screw **2100,2200** may include an additional polymer material anywhere along the length of the screw **2100,2200** either coupled to an interface portion or as an alternating section of material. This additional polymer material may also be deformed, once the screw **2100,2200** is placed in the bone, to provide further fixation of the screw **2100,2200** and further compression of the fracture.

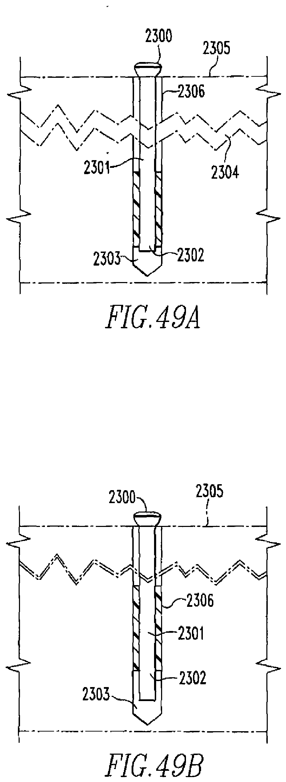

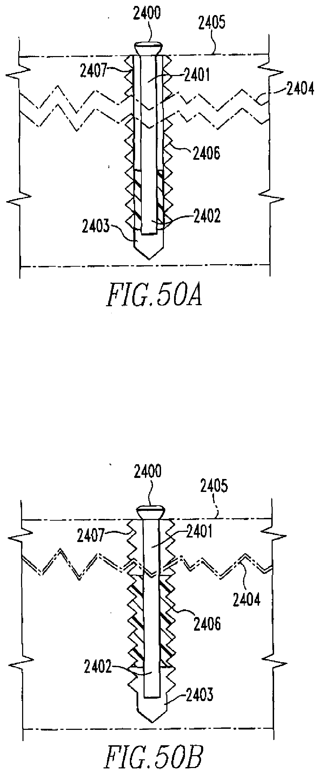

Figs. 49A and 49B show an alternative method of compressing a fracture. In Figs. 49A and 49B, an internal fixation device **2300,** which includes a polymer material **2301** coupled to a shaft **2302** of the device **2300,** is located in a hole **2303** that has been drilled through a fracture **2304** of a bone **2305.** The polymer material **2301** is then provided with energy, via any of the methods described above or any other method known to one of ordinary skill in the art, to deform the material **2301,** thereby fixating the device **2300** to the bone **2305** and compressing the fracture **2304,** as shown in Fig. 49B. Figs. 50A and 50B provide a similar method of compressing the fracture, but rather than being located in a drilled hole, the internal fixation device **2400** is located in a tapped hole **2403.** The sides **2406** of the tapped hole **2403** have serrated edges **2407,** rather than the substantially smooth sides **2306** that are present in the drilled hole **2303.** Upon deformation of the polymer material **2401,** the material **2401** may deform to fit within these serrations **2407.** With the material **2401** being coupled to the shaft **2402** and deformed to fit within the serrations **2407,** the shape of the device/material combination resembles that of a threaded fastener and therefore, removal of the device **2400** from the hole **2403** may be done in a manner similar to the removal of a threaded fastener.

The polymer material **2401** may be located anywhere on the internal fixation device **2300,2400.** In addition, the holes **2303,2403** may be formed by a drill and a tap, but may be formed by any other device known to one of ordinary skill in the art for making drilled and tapped holes. Furthermore, rather than being drilled or tapped, the holes may be broached or formed in any other manner known to one of ordinary skill in the art.

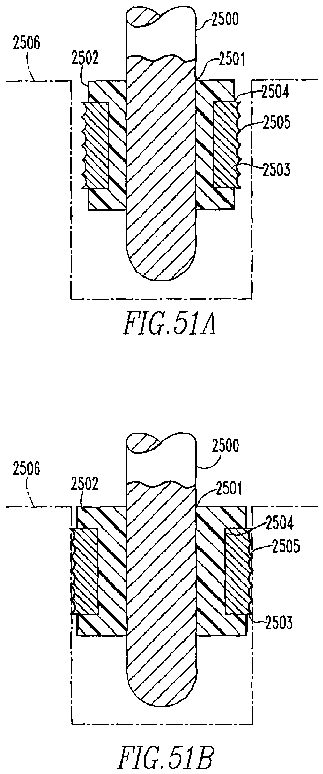

Figs. 51A and 51B show an internal fixation device **2500** having an interface portion **2501,** a polymer material **2502** coupled to the interface portion **2501,** and at least one feature, such as protrusions **2503,** that are coupled to a surface **2504** of the polymer material **2502.** The protrusions **2503** may be coupled to the polymer surface **2504** via a variety of methods, such as an interference fit between the polymer **2502** and the protrusions **2503,** adhesion of the protrusion **2503** to the polymer **2502,** or any other method known to one of ordinary skill in the art. In addition, the number of protrusions **2503** present on the polymer material **2502** may vary. As shown in figures 51A and 51B, the protrusions **2503** include serrations **1505** located on an outside surface of the protrusions **2503.** The serrations **2505** provide multiple contact points to increase the friction between the polymer material **2502** and the bone **2506,** thereby providing increased fixation between the device **2500** and the bone **2506** and substantially reducing the possibility of axial and torsional rotation of the device **2500.** The protrusions **2503** are selected from a group that includes a metal material, a non-metal material, a polymer material, and combinations thereof and may be of any shape or size. If a polymer material is used for the protrusions **2503,** the polymer material may include a resorbable or non-resorbable polymer material. In addition, surface features other than serrations may be used to provide multiple contact points and increase the friction between polymer material **2502** and the bone **2506.**

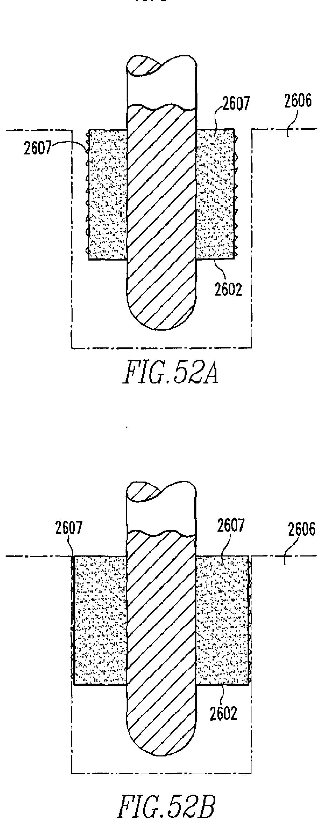

Use of the protrusions **2503** in Figs. 51A and 51B may be eliminated by including a particulate material **2607** within or on an outer surface of a polymer material **2602,** as shown in Figs. 52A and 52B. The particulate material **2607** may include a ceramic material, a crystalline polymer, or any other type of material that would provide the polymer material **2602** with multiple contact points to increase the friction between the polymer material **2602** and the bone **2606.**

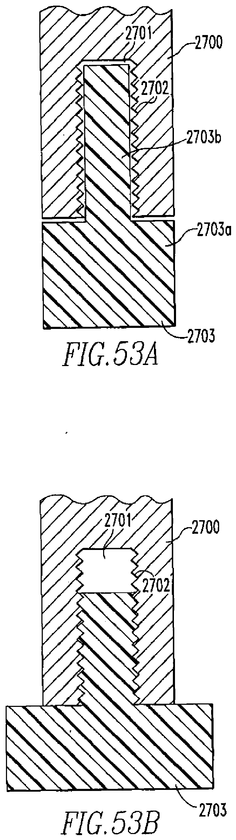

Fig. 53A shows an internal fixation device **2700,** such as an intramedullary nail, having a channel **2701** partially extending the length of the device **2700.** The channel **2701,** which includes a threaded inner surface **2702,** may be of a variety of lengths and widths. In addition, the inner surface **2702** of the channel **2701** may include a feature other than threads or may be smooth. A polymer material **2703,** including a body **2703a** having a stem portion **2703b,** is coupled to the device **2700,** such that the stem portion **2703b** is located within the channel **2701.** As shown in Fig. 53B, once the device **2700** is inserted into a bone, the polymer material **2703** is deformed, via one of the methods described above or another method known to one of ordinary skill in the art, to expand the material **2703** radially and fixate the device **2700** to bone. The stem portion **2703b** of the material **2703** also expands radially to engage the threaded inner surface **2702** and fixate the material **2703** to the device **2700.**

In some applications, the stem portion 2703b may include threads configured for engagement with the threads on the inner surface 2702 when the stem portion 2703b is disposed within the channel 2701. In some further applications, the outer surface of the device 2700 may include surface features, such as the holes, slots, threads, ribs, and engravings shown in Figs. 17-21 above or other surface features known to one of skill in the art, which may extend between the outer surface and the channel 2701. In addition to allowing formation of bonds between the polymer material 2703 and the inner 2702 and outer surfaces, once the polymer material 2703 is provided with energy, these surface features help the polymer material 2703 engage the device 2700 to provide support for axial and torsional loading and to substantially reduce motion in those directions after the device 2700 has been placed in bone. It is within the scope of this disclosure that the channel 2701 may extend the full length of the device 2700. It is also within the scope of this disclosure that the body 2703a may extend over and around the outer surface of the device 2700 and, in some applications, may extend around the surface features described above.

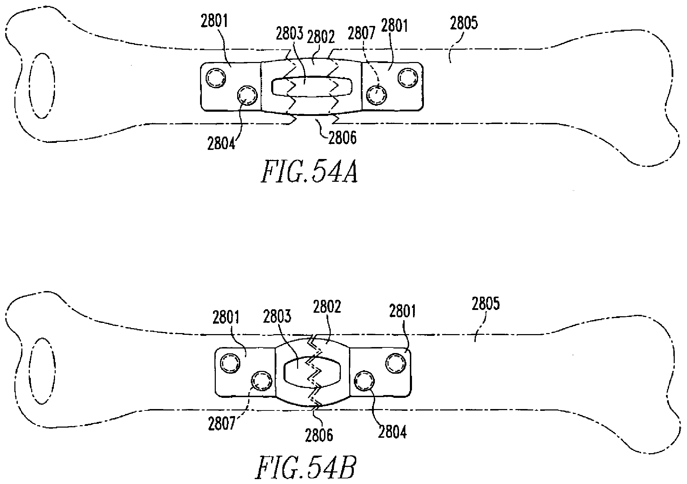

Fig. 54A shows a bone plate **2800** that includes two end portions **2801** and a middle portion **2802.** The middle portion **2802** includes a center opening **2803** and a polymer material that has shape memory qualities or a shape memory alloy material. Both end portions **2801** include at least one hole **2804** and a metal, a non-metal, or a polymer material that does not have shape memory qualities. The plate **2800** is placed on a fractured bone **2805,** such that the middle portion **2802** is located adjacent to the fracture **2806,** and fixated to the bone **2805** by inserting fasteners **2807** through the holes **2804** of the end portions **2801** and into the bone **2805.** The fasteners **2807** include locking screws, non-locking screws, rods, pins, or any other fastener that may be used to fixate the plate **2800** to the bone **2805.** Once the plate **2800** is fixated to the bone **2805,** the middle portion **2802** of the plate **2800** is provided with energy to deform the middle portion **2802** and compress the fracture **2806,** as shown in Fig. 54B. The number and location of holes **2804** on the end portions **2801** may vary.

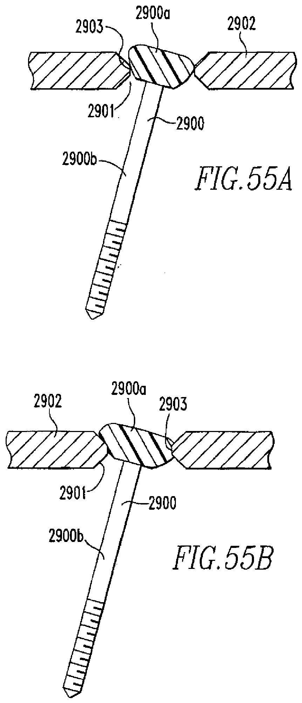

Fig. 55A shows a fastener **2900** located in an opening **2901** of a fixation device **2902.** The fastener **2900** includes a head **2900a** and a shaft **2900b.** The head **2900a,** which includes a shape memory polymer material, rests within an inner wall **2903** of the opening **2901.** Once the fastener **2900** is inserted through the opening **2901** and into a bone, the head **2900a** is provided with energy, via one of the methods described above or another method known to one of ordinary skill in the art, to engage the head **2900a** with the inner wall **2903** and further fixate the fixation device **2902** to the bone. The fastener **2900** includes a locking screw, a non-locking screw, a rod, a pin, or any other fastener that may be used to fixate the fixation device **2902** to the bone. In addition, the head **2900a** of the fastener **2900** may be of a variety of shapes and sizes. Furthermore, the inner wall **2903** of the opening **2901** includes a v-shaped cross section, but may include a variety of other surface features, such as ridges, threads, protrusions, or other features that would provide engagement with the head **2900a,** upon deformation, and further fixate the device **2902** to the bone.

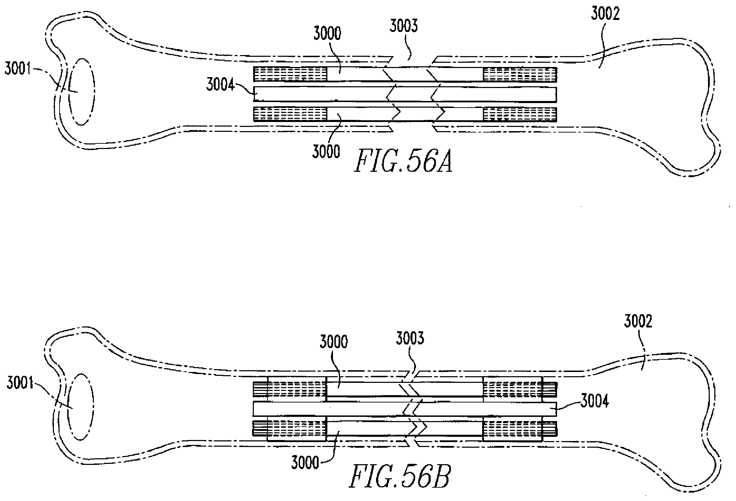

Figs. 56A and 56B show first internal fixation devices **3000,** shown as rods, which have been inserted into the intramedullary canal, through the entry point **3001** at the one end of the bone **3002,** and placed across the fracture site **3003.** However, mixed with these devices **3000** is a second internal fixation device **3004** that is made entirely out of a high strength resorbable polymer material and does not have shape memory qualities. The second internal fixation devices **3004** provide reinforcement to the first internal fixation devices **3000.** The number of first **3000** and second **3004** internal fixation devices varies and includes as many as is necessary to fill the canal. Alternatively, instead of having first internal fixation devices that include a shape memory polymer material coupled to the ends of the devices, the first internal fixation devices may be entirely composed of a shape memory polymer material. In addition, the second internal fixation devices **3004** may include a metal or non-metal material, rather than a high strength resorbable polymer material.

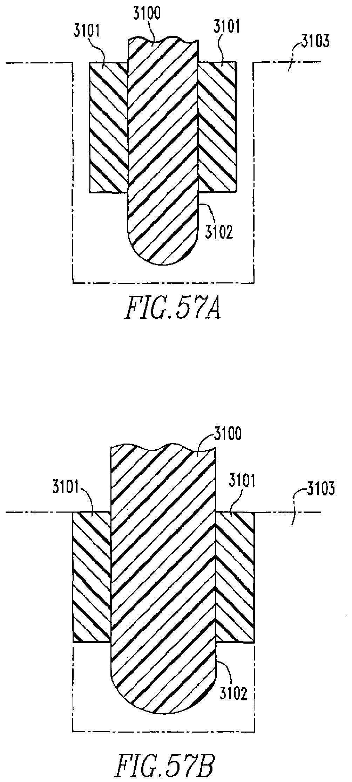

Fig. 57A shows an internal fixation device **3100** that includes protrusions **3101** on an outer surface **3102** of the device **3100.** The protrusions **3101** extend the length of the device **3100** and may include a polymer material that does not include shape memory qualities, a metal material, or a non-metal material. The internal fixation device **3100** is composed completely of a shape memory polymer material. The number and location of the protrusions **3101** on the outer surface **3102** of the device **3100** varies. In use, the device **3100** is inserted into a bone **3103** and then provided with energy to deform the device **3100** and allow engagement of the protrusions with the bone **3103,** thereby fixating the device **3100** to the bone **3103,** as shown in Fig. 57B. The protrusions **3101** may extend less than the length of the device **3100** and may include serrations or other surface features that would allow the protrusions **3101** to further engage the bone **1903.**

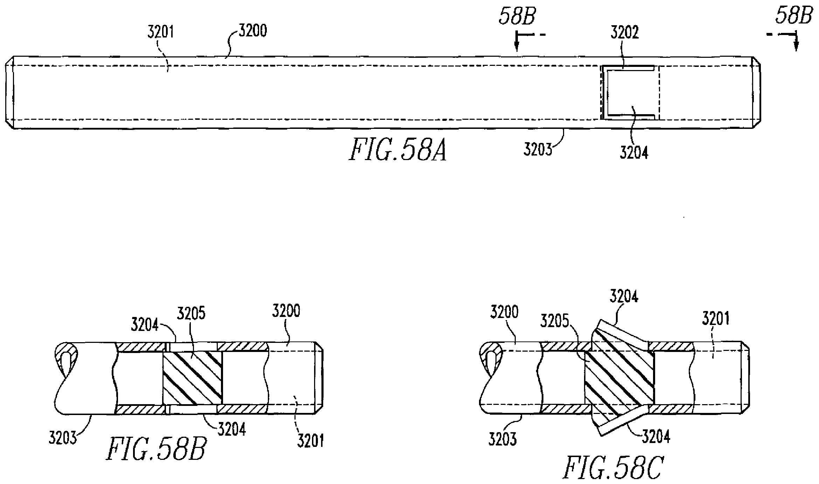

Fig. 58A shows an internal fixation device **3200,** such as an intramedullary nail, that includes a cannulated inner portion **3201** and at least two C-shaped channels **3202** located opposite each other on an outer portion **3203** of the internal fixation device **3200,** wherein the channels **3202** include tabs **3204.** The tabs **3204** include a material, such as elastic, that would allow the tabs **3204** to open outward, away from the outer portion **3203** of the device **3200,** upon deformation of a polymer material and close, after resorption of the material, as described below. As shown in Figs. 58B and 58C, a resorbable shape memory polymer material **3205** is located within the inner portion **3201** of the device **3200** and between the C-shaped channels **3202.** The material **3205** may be held within the inner portion **3201** via an interference fit between the material **3205** and the inner portion **3201** or by another method known to one of ordinary skill in the art. In use, the device **3200** is inserted into a bone and the polymer material **3205** is provided with energy, via one of the methods described above or another method known to one of ordinary skill in the art, to expand the material **3205** radially and open the tabs **3204** outwardly away from the outer portion **3203** of the device, as shown in Fig. 58C, and toward the bone. In this manner, the tabs **3204** engage the bone and provide fixation of the device **3200** to the bone. Upon resorption of the polymer material **3205,** the tabs **3204** would move back towards the device **3200,** thereby allowing the device **3200** to lose fixation with the bone and provide the dynamization that is required, as described above. The number and location of channels **3202** may vary. In addition, the cannulated inner portion **3201** may be of a variety of lengths and widths and the polymer material **3205** may be in a variety of shapes and sizes.

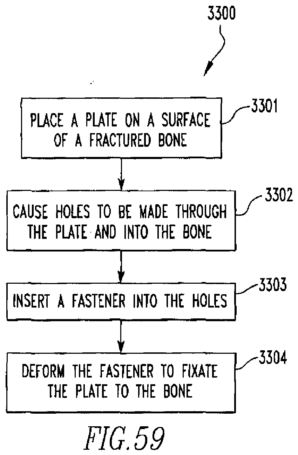

Fig. 59 shows a method **3300** of fixating a bone plate to a fractured bone. The method includes placing a bone plate on a surface of a fractured bone **3301,** causing holes to be made through the plate and into the bone **3302,** inserting a fastener into the holes **3303,** and deforming the fastener to fixate the plate to the bone **3304.** The bone plate would not include holes prior to placing the plate on the bone, but may include suggested areas in which holes could be made. For example, the plate may include indentations, notches, or circled areas on an outer surface of the plate that represent recommended areas in which to create holes. The holes may be caused by drilling, tapping, broaching, or any other method known to one of ordinary skill in the art for creating holes in a bone plate and bone. The fastener includes shape memory polymer material and may be composed entirely of this material or be composed of alternating sections of polymer material having shape memory qualities and polymer material that does not have shape memory polymer material. For example, the fastener may be in the form of a cylindrical rod and the portion of the rod that is housed in the holes of the plate and the bone may be composed of non-shape memory polymer material, but the portion that is located outside of the holes may be composed of shape memory polymer material. In this example, the portion located outside of the holes would be provided with energy to deform the portion and fixate the plate to the bone.

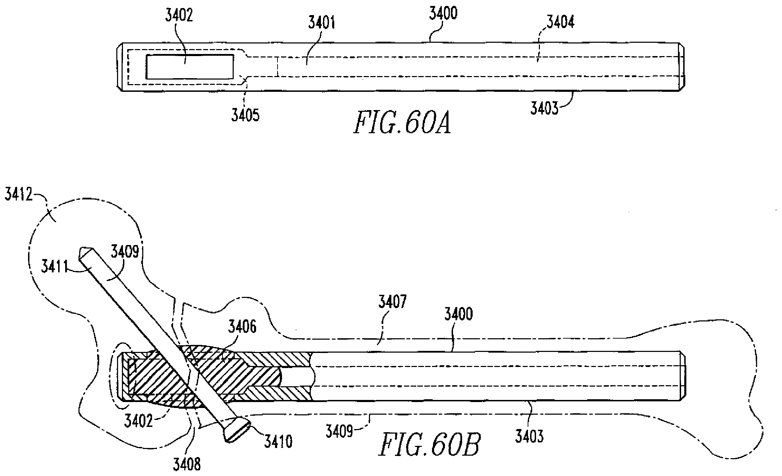

Fig. 60A shows an internal fixation device 3400, such as an intramedullary nail, that includes a cannulated inner portion 3401 and an opening 3402 on an outer portion 3403 of the device 3400. The cannulated inner portion 3401 includes a first section 3404 and a second section 3405, wherein the second section 3405 includes a larger diameter than the first section 3404. Located within the second section 3405 is a shape memory polymer material 3406. The opening 3402 is located adjacent to the second section 3405 and the polymer material 3406. In use, the device 3400 is inserted through an intramedullary canal of a fractured bone 3407, such that the second section 3405 and the opening 3402 are placed across the fracture 3408. A fastener 3409, having a head 3410 and a shaft 3411, is inserted through the outer surface 3409 of the bone 3407, through the opening 3402, and into the inner surface 3412. In this manner, the fastener 3409 stabilizes and reduces the fracture 3408. Once the fastener 3409 has been located in the bone 3407, the polymer material 3406 is then provided with energy to deform the material 3406 and further fixate the fastener 3409 to the device 3400. The first and second sections 3404, 3405 of the cannulated inner portion 3401 may be of a variety of lengths and widths and the polymer material 3406 may be in a variety of shapes and sizes. In addition, the opening 3402 is of any diameter that is larger than the diameter of the shaft 3411 of the fastener 3409.

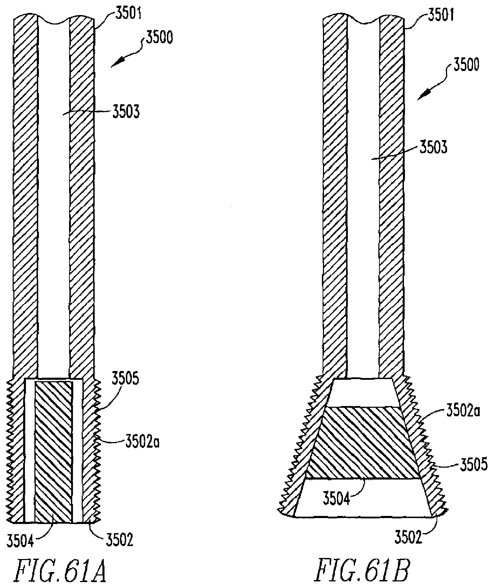

Fig. 61A shows a cross-sectional view of an internal fixation device 3500, such as an intramedullary nail, having a proximal portion 3501, a distal portion 3502, and a central channel 3503 extending an entire length of the device 3500. A polymer material 3504 is located within the channel 3503 at the distal portion 3502 of the device 3500. The distal portion 3502 is hinged or tabbed to allow expansion of the distal portion 3502 upon expansion of the polymer material 3504, as will be further described below. In addition, the distal portion 3502 includes at least one feature, such as protrusions 3505, on a surface 3502a of the distal portion 3502. As shown in Fig. 61B, once the device 3500 is inserted into a bone, the polymer material 3504 is deformed, via one of the methods described above or another method known to one of ordinary skill in the art, to expand the material 3504 radially, thereby expanding the hinged distal portion 3502 outward to engage the bone and fixate the device 3500 to bone. The distal portion 3502 of the device 3500 may be coupled to the proximal portion 3501 via a hinge, tab, or any other coupling device that is made from biocompatible material. Alternatively, the distal portion 3502 may have an area that is thinner than the rest of the device 3500 and allows the distal portion 3502 to expand outward and engage bone.

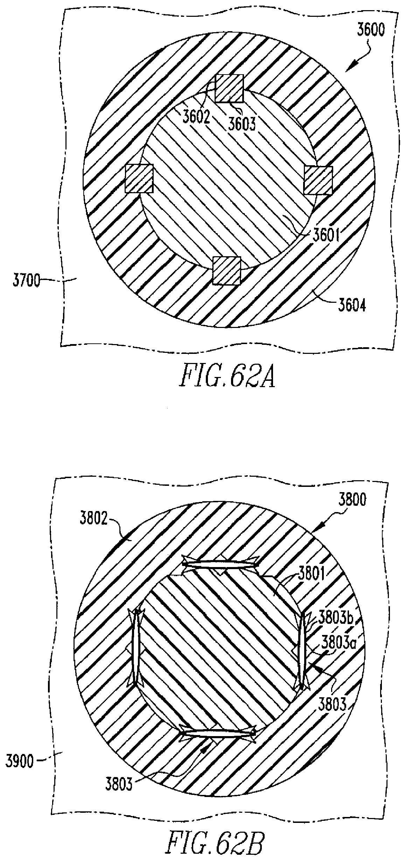

Fig. 62A shows a cross-sectional end view of a construct 3600 including an internal fixation device 3601 having channels 3602, rods 3603 disposed within the channels 3602, and a sleeve 3604 of shape memory polymer material, similar to the sleeves described above, disposed over the device 3601 and the rods 3603. The construct 3600 is disposed within bone 3700 with the material 3604 having been supplied with energy, via a process described above or another process known to one of skill in the art, to deform the material 3604 and fixate both the device 3601 to the bone 3700 and the material 3604 to the rods 3603.

The device 3601 includes a metal material, but may include a non-metal material. The channels 3602 may be formed in the device 3601 via a machining process or other process known to one of skill in the art. The rods 3603 may include a metal material or another material that would make the rods 3603 solid enough in construction to substantially reduce deformation of the rods 3603 when the construct 3600 is inserted into the bone 3700 and the material 3604 is activated. The channels 3602 and rods 3603 may be continuous and extend a partial or full length of the device 3601 or they may be non-continuous and separated along a full or partial length of the device 3601. In addition, it is not necessary for the channels 3602 and the rods 3603 to extend the entire diameter of the device 3601 and the number of channels 3602 and rods 3603 will vary. In some applications, the rods 3603 may be textured to improve the integration of the material 3604 into the rods 3603, therefore allowing for increased fixation of the material 3604 to the rods 3603.

Fig. 62B shows a cross-sectional end view of a construct 3800 similar to the construct 3600 shown in Fig. 62A. The construct 3800 includes an internal fixation device 3801, a sleeve 3802 of shape memory polymer material, similar to the sleeves described above, and components 3803 located between the device 3801 and the sleeve 3802. The components 3803 include barbs 3803a on the outer surface 3803b of the components 3803 for purposes that will be described below. The construct 3800 is disposed within bone 3900 with the material 3802 having been supplied with energy, via a process described above or another process known to one of skill in the art, to deform the material 3802 and fixate both the device 3801 to the bone 3900 and the material 3802 to the components 3803.

The device 3801 includes a non-metal material, such as a polymer material, but may include other non-metal or metal materials that allow the barbs 3803a to be embedded within the device 3801. The components 3803 may include a metal material or another material that would make the components 3803 solid enough in construction to allow the components 3803 to be embedded within the device 3801 and the sleeve 3802 when the construct 3800 is inserted into the bone 3900 and the material 3802 is activated. The components 3803 may be continuous and extend a partial or full length of the device 3801 or they may be non-continuous and separated along a full or partial length of the device 3801. In addition, it is not necessary for the components 3803 to extend the entire diameter of the device 3801 and the number of components 3803 will vary. In some applications, the components 3803 may be textured to improve the integration of the material 3802 into the components 3803, therefore allowing for increased fixation of the mat material 3802 to the components 3803.

As stated throughout this disclosure, internal fixation devices are used for fracture reduction, fixation or stabilization, and compression. For devices such as intramedullary nails, rods, and pins, fracture reduction and stabilization may occur via a method according to the following steps: creation of an entry portal at a location along the bone, provisional reduction of a fracture via the use of a reducer or other tool known to one of ordinary skill in the art for reducing fractures, insertion of the internal fixation device through the entry portal and placement of the device across the fracture, fixation of one side of the fracture by insertion of at least one fastener through the device or, as described above, expansion of a shape memory polymer material, reduction of the fracture via the application of pressure on the device or on the other side of the fracture that has not been fixated, and fixation of the other side of the fracture by insertion of at least one fastener through the device or, as described above, expansion of a shape memory polymer material. It is within the scope of this disclosure that the fixation and reduction steps may occur in a different order. For example, both sides of the fracture may be fixated before the fracture is reduced. This is especially true through, as described above, the use of a device that can compress the fracture via the use of an expandable polymer material.

For a device, such as a bone plate, fracture reduction and stabilization may occur via a method according to the following steps: reduction of the fracture, placement of a plate across the fracture via the use of an instrument or provisional fixation device, such as a forceps or pins/wires, to hold the plate to the bone while the plate is being fixated to the bone, placement of at least one non-locking or locking fastener through a hole in the plate and a hole on one side of the fracture, placement of at least one nonlocking or locking fastener through a hole in the plate and a hole on another side of the fracture and compression of the fracture either manually, via the use of compression screws, or with a device that uses expandable polymer material, as described above. It is within the scope of this disclosure that the holes may be made in the plate and the bone after the plate is placed across the fracture. The holes may be created through the use of a drill, a tap, a broach, or another instrument known to one of ordinary skill in the art for creating holes in the plate and bone. It is also within the scope of this disclosure that the fasteners may be fasteners that lock to the plate via the use of expandable material on either the head of the fastener or on the inner wall of the plate or bone holes, as described above. Also, multiple fasteners, used on one or both sides of the fracture, may be used to fixate the plate to the bone. It is within the scope of this disclosure that the fixation and reduction steps may occur in a different order. For example, both sides of the fracture may be fixated before the fracture is reduced. This is especially true through, as described above, the use of a device that can compress the fracture via the use of an expandable polymer material.

When the polymer material includes alternating segments of a shape memory polymer material and a non-shape memory polymer material, each shape memory segment can be individually provided with energy at separate time intervals in order to gradually cause straightening, bending, shortening, or lengthening of the material. For example, a first segment of shape memory polymer material can be activated to expand and shorten. This causes a first length of the material attached to the device to shorten. After a period of time, a second segment of shape memory polymer is activated to cause further shortening of material. This is beneficial for treatments requiring long-term remodeling, such as scoliosis or other deformities.

The present disclosure is directed to the use of electrical and thermal energy sources to heat the polymer material and deform it. However, the polymer material could be deformed via other methods known to those of ordinary skill in the art, including, but not limited to the use of force, or mechanical energy, a solvent, and /or a magnetic field. Any suitable force that can be applied either preoperatively or intra-operatively can be used. One example includes the use of ultrasonic devices, which can deform the polymer material with minimal heat generation. Solvents that could be used include organic-based solvents and aqueous-based solvents, including body fluids. Care should be taken that the selected solvent is not contra indicated for the patient, particularly when the solvent is used intra-operatively. The choice of solvents will also be selected based upon the material to be deformed. Examples of solvents that can be used to deform the polymer material include alcohols, glycols, glycol ethers, oils, fatty acids, acetates, acetylenes, ketones, aromatic hydrocarbon solvents, and chlorinated solvents. Finally, the polymer material could include magnetic particles and deformation could be initiated by inductive heating of the magnetic particles through the use of a magnetic field.

It is also within the scope of this disclosure to include a pressure sensor within the sleeve of shape memory polymer material that is coupled to the internal fixation device. This pressure sensor may communicate with the hand-held battery powered device described above or another power control unit to provide the user with a measurement of the amount of force that exists between the bone and the internal fixation device when the sleeve is provided with energy and expands against the inner wall of the bone. The force may be monitored and regulated based on the measurement. In addition, a thermocouple may be placed on the inner wall of the sleeve to measure the temperature of the polymer material as it is provided with energy. The thermocouple may communicate with the hand-held battery-powered device to allow the user to know when the temperature of the material has gone above the glass transition temperature of the material and the material has therefore begun to deform. The thermocouple would also be advantageous in monitoring the temperature of the material during the application of energy such that the user would be able to substantially reduce the possibility of the temperature reaching the melting temperature of the material.

Within the scope of this disclosure is also the possibility of the sleeve of shape memory polymer being fixated to the interface portion of the internal fixation device via the use of biocompatible glue, rather than relying on the above-described properties or textures on the surface of the interface portion to provide fixation of the sleeve to the device.

### EXAMPLE ONE

The present example provides a fabrication process for the internal fixation device of the present disclosure, a method of fixating the device to bone, and test results on the fixation strength of the device.

Sleeves of a polymer composite were manufactured using a copolymer and a filler material. Specifically, 600 g of a copolymer of poly L-lactic acid (PLLA) and poly D-lactic acid (PDLA) having a glycolide component was vacuum dried at a temperature of about 50°C and a pressure of about 10 millibars for 48 hours. The ratio between the lactide unit and the glycolide unit was 85:15. 300 g of a filler material, namely calcium carbonate, were placed in a 1000 ml glass jar and vacuum dried at about 150°C and a pressure of about 10 millibars for 48 hours. A dry blend was then produced by mixing the copolymer and calcium carbonate. This blend was then compounded in a prism twin screw extruder to form pellets of a copolymer/calcium carbonate composite. These pellets were placed into a cylindrical mould that was sealed at one end with a plug. A pressure of about 20 MPa was applied to the pellets via a plunger and the temperature of the mould was raised to a level that was sufficient to melt the pellets, or about 200°C. The temperature was maintained at this level for 20 minutes. The mould was then cooled to room temperature and the pressure was released by removal of the plunger. The plug was removed from the mould and a billet of the polymer composite material was pressed out of the mold. The billet was die drawn to produce a final rod of material having a diameter of about 8 mm.

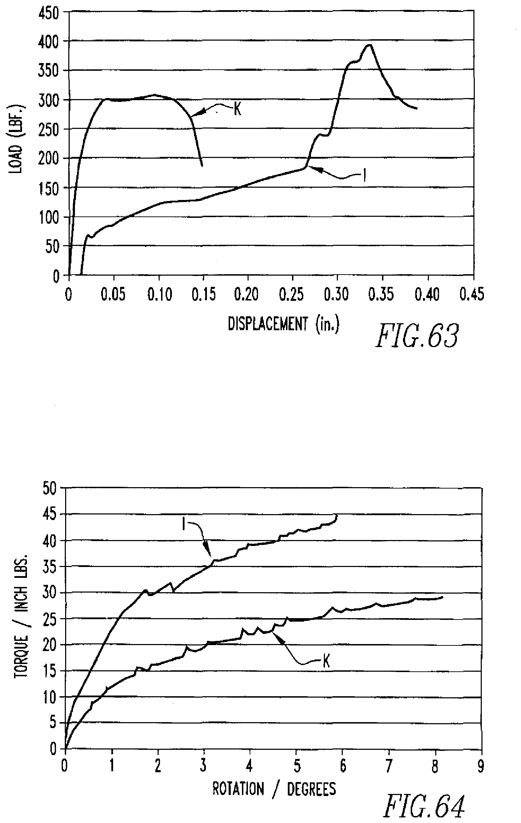

Interface portions corresponding to Figs. 2I and 2K were machined onto 2 steel rods, each rod having an outside diameter of 0.375 inches. The length of the interface portion was about 0.75 inches. The above-mentioned sleeves of polymer composite, also having an outer diameter of 0.375 inches, were machined and placed over the interface portion of each rod. The interface portion/polymer composite area of the rods were placed into a 7/16 inch hole that had been drilled into a block of 20 lb synthetic bone. The rod and synthetic bone combinations were then immersed in a water batch at 50°C for 1 hour and then removed and allowed to dry at room temperature. Immersion of the rod and synthetic bone combinations into water caused the polymer material to deform and fixate the rod to the bone. The fixation of the rods was tested by clamping the synthetic bone and testing the pullout and torque strengths of the rods by using a loading rate of 0.1 inches/min and 10°/min, respectively. Results for the pullout test and the torsion test are given in Figs. 63 and 64, respectively. The test results for the interface portion corresponding to Fig. 2I are represented as "I" in the figures and the test results for interface portion corresponding to Fig. 2K are represented as "K" in the figures.

This example shows that an internal fixation device, having a shaped interface portion and a polymer material coupled to the interface portion, can be fixated to bone by providing energy to the polymer material and thereby causing the material to deform and engage the bone. In addition, tests performed on the fixation strength of the device show that the device is able to withstand a variety of loading rates without becoming dislodged from the bone. As mentioned above, fixation of these devices to bone via use of the polymer material, rather than mechanical fasteners, such as screws and pins, provide significant advantages to both the surgeon and the patient. In addition, adequate fixation of the device to bone will be beneficial in maintaining a compressive load across a fracture site over a longer period of healing.

### EXAMPLE TWO

Two Delrin rods were used to simulate a fractured bone. Ends of the rods were placed adjacent to each other with the point at which the ends of the two rods met being defined as the simulated fracture point. Each rod had a diameter of about .75 inches, a length of about 4.3 inches, and a 7 mm diameter through hole that extended the entire length of each rod.

Fiber reinforced composite rods were then manufactured. PLLA fiber was first made by taking PLLA granules with a nominal intrinsic viscosity of 3.8 and extruding the granules into a fiber. A single screw extruder fitted with a gear pump and a 2 mm spinneret die was used. The extruder also had a provision for air cooling. The extruded fiber was batched on spools for the next processing step. Subsequently, the fiber was progressively stretched at elevated temperatures to produce a final diameter of ca. 100 microns and a draw ratio between about 8 and about 15. The final molecular weight of the drawn fiber was between about 290,000 g/mol-1 to about 516,000 gmol-1. The resultant fiber had an average tensile strength of greater than about 800 MPa.

Composites were then made using an 85:15 co-polymer of PDLLA and PGA with a 35 % weight addition of calcium carbonate (CaCO3) as the matrix material. The drawn poly (L-lactide) fibers were then wound around a support frame of parallel bars that were held a constant distance apart. For each sample the fiber was wrapped 75 times around the support frame, resulting in 150 fibers in each composite. The matrix was dissolved in a solvent, methyl acetate, at 10% wt/vol of solvent. The solvent/polymer mixture was then coated onto the fibers. The composite was then placed in a vacuum oven at 40°C for 12 hours to remove the solvent.

The composite was then placed in a cylindrical mold with an internal diameter of about 2 mm and heated to 165°C. This temperature is used to melt the matrix material to allow it to flow and consolidate the composite. Once thermal equilibrium was reached, slight tension was applied to the fibers to align them in the mold. The mold was then closed completely to consolidate the fibers and the matrix. The closed mold was then maintained at 165°C for up to 5 minutes and then removed from the heated press and placed between cool metal blocks to cool the composite down to room temperature to allow tension to be released from the fibers. This resulted in lengths of composite rod with an approximate diameter of 2 mm.

**A** construct was then made by placing the rods within the simulated bone to extend across the fracture point and gluing the rods into place using a thermoset matrix material, such as a degradable 2 part polyurethane material obtainable from PolyNovo Biomaterials Ltd. located in Victoria, Australia. The polyurethane was inserted into the simulated bone via the use of a syringe and allowed to cure overnight. The polyurethane material stabilizes the rods and fixates the rods to the bone, thereby stabilizing the fracture.

The bending strength and shear strength of the construct were then tested. The bending strength was tested using a cantilever test, in which one half of the construct was held rigid while a load was applied to the other half of the construct at a point that was located about 50 mm away from the fracture location. The construct was loaded at a rate of 5 mm/min to deflect the one half of the construct at a 10° angle relative to the other half. The force required to deflect the construct 10° was 19 lbs. The shear strength was tested by clamping both halves of the construct to displace the two halves 2 mm relative to each other at a rate of 10 mm/min. The force required to shear the construct 2 mm was 362 lbs.

### EXAMPLE THREE

A die-drawn PLDLA(70/30) rod containing 35% wt/wt of calcium carbonate was machined into a plug having a diameter of 13 mm and a length of 25mm. The plug included a stem having a length of 20 mm and a diameter of 8 mm. The plug was similar in construction to the polymer material shown in Figs. 53A-53B. A hole of 3/16 inch diameter was drilled through the centre the plug. Once machined into these dimensions, a 40mm length of steel tubing, referred to as a metal sleeve, was inserted into the hole.

A stainless steel tubing (8mm ID/12 mm OD) was generated such that one end of the tubing was profiled to have slots, 3 (6mm semicircle slots) and 3 (4X8mm elliptical slots), and the other end was machined to have 3 flat surfaces suitable for an instron to grip. The stem of the plug with metal sleeve was inserted into the end (containing the slots) of the stainless steel tubing, to form a construct, and this construct was placed into the canal of a section of femur, approximately 50mm in length and 17mmX16mm in diameter. The bone was left to equilibrate to room temperature.

A heating probe (4mm diameter), which was connected to and controlled by a DC power supply, was inserted into the metal sleeve and the power supply was switched on (18 Volts and a little over 1 Amp). Once the probe reached the desired temperature, a timer was started. At a set time point, about 15 minutes, the heating probe was removed from inside the sleeve and the bone containing the polymer plug was immersed in cold water.

Once removed from the cold water, a pushout test was carried out using an Instron 5566 with 10kN load cell and Bluehill software program. The stainless steel tubing was clamped in the stationary grip at the top of the test frame and the bone with plug was placed on a plate on top of the crosshead. During testing, the plate on the crosshead was raised upwards at a rate of 1mm/minute. The push out forces for the bone/tube construct was measured and found to 1353N.

### EXAMPLE FOUR

The experiment of example three was repeated with a section of femur having a canal of approximately 50mm in length and 18.7mmX 17.6mm in diameter. A pushout force of 961N was recorded.

FIG. 66 illustrates a fastener for locating a shape memory material. FIG. 66 illustrates a bone 4010, a shape memory material 4012, a fastener 4014, and an intramedullary cavity 4016. As examples, the fastener 4014 may be a pin, a wire, a Kirschner wire, a screw, a dowel, a spike, or a suture. The fastener 4014 may be placed proximate the fracture site. In some embodiments, the fastener 4014 may be placed within the fracture site. The fastener 4014 allows for adequate expansion on each side of a fracture site and to achieve desired placement of the expanded shape memory material.

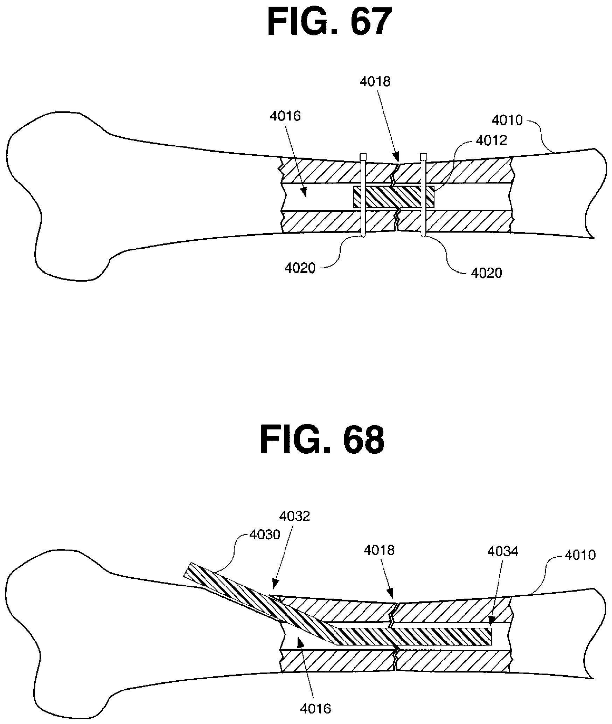

FIG. 67 illustrates a plurality of fasteners for locating a shape memory material. FIG. 67 illustrates a bone 4010, a shape memory material 4012, a fracture site 4018, a plurality of fastening elements 4020, and an intramedullary cavity 4016. As examples, the fastening element 4020 may be a pin, a wire, a Kirschner wire, a screw, a dowel, a spike, or a suture. In FIG. 67, there are two fastening elements 4020, but any number of fastening elements may be used. The fastening elements 4020 allow for adequate expansion on each side of a fracture site and achieve desired placement of the expanded shape memory material.

FIG. 68 illustrates a cut-to-length shape memory material. FIG. 68 illustrates the bone 4010, the intramedullary cavity 4016, the fracture site 4018, and a cut-to-length shape memory material 4030. In this embodiment, an opening 4032 is created into a portion of the bone 4010. The cut-to-length shape memory material 4030 is inserted into the opening 4032 and fed into the intramedullary cavity until a distal end portion 4034 is adequately placed relative to the fracture site 4018. Thereafter, the cut-to-length shape memory material 4030 is energized to allow for adequate expansion on each side of the fracture site 4018. Finally, the excess shape memory material is removed at the opening 4032.

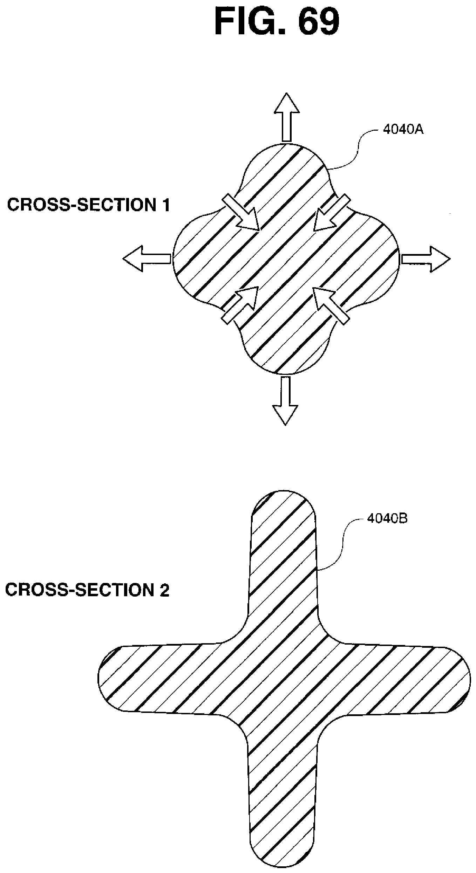

FIG. 69 illustrates a first and a second cross section of a shape memory material. FIG. 69 illustrates a shape memory material having a non-expanded cross-section 4040A and an expanded cross-section 4040B. The cross-sections 4040A, 4040B are constructed and arranged to allow for adequate expansion on each side of a fracture site and achieve desired placement of the expanded shape memory material. In the depicted embodiment, the cross-section 4040A is generally cylindrical and the cross-section 4040B is generally triangular. In some embodiments, the cross-section 4040B may have a plurality of lobes. Although the shape memory material changes in cross-section, the overall length is substantially maintained.

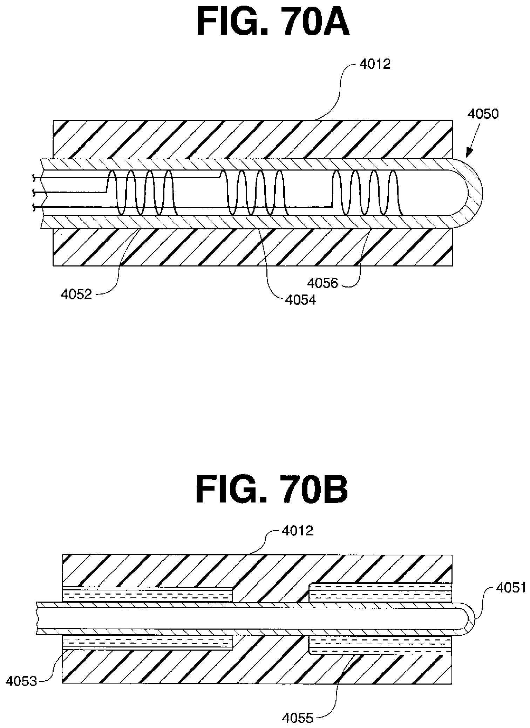

FIG. 70A illustrates a heating device having a plurality of heating elements. FIG. 70A illustrates the shape memory material 4012 having a cannulation and a heating device 4050 placed within the cannulation. The heating device 4050 has a plurality of heating elements. In the depicted embodiment, there are three heating elements 4052, 4054, 4056 but any number of heating elements may be used. The heating elements 4052, 4054, 4056 may be selectively engaged such that portions of the shape memory material may be energized at different times. For example, it may be desirable to energize the middle of the shape memory material before energizing the ends or vice versa. In some embodiments, the winding density of the heating elements may be varied relative to one another to achieve different rates of heating. In this manner, the heating elements may be used to control deployment of the shape memory material.

In some embodiments, other forms of energy may be used to control deployment of the shape memory material. Examples of other forms may include electromagnetic or acoustic energy. The energy may be selectively targeted at portions of the shape memory material to control deployment. For example, ultrasound energy first may be delivered to the fracture site to deploy the shape memory material and then targeted towards the ends of the shape memory material to achieve full expansion.

FIG. 70B illustrates a device having shape memory material, a heating element and a plurality of insulation elements. FIG. 70B illustrates the shape memory material 4012 having a cannulation and a heating element 4051 placed within the cannulation. The device also has a plurality of insulation elements. As examples, the insulation elements may be a coating or an air gap. In the depicted embodiment, there are two insulation elements 4053, 4055 but any number of insulation elements may be used. The insulation elements 4053, 4055 provide different levels of thermal conductivity such that portions of the shape memory material may be energized at different times. For example, it may be desirable to energize the middle of the shape memory material before energizing the ends or vice versa. In this manner, the insulation elements may be used to control deployment of the shape memory material.

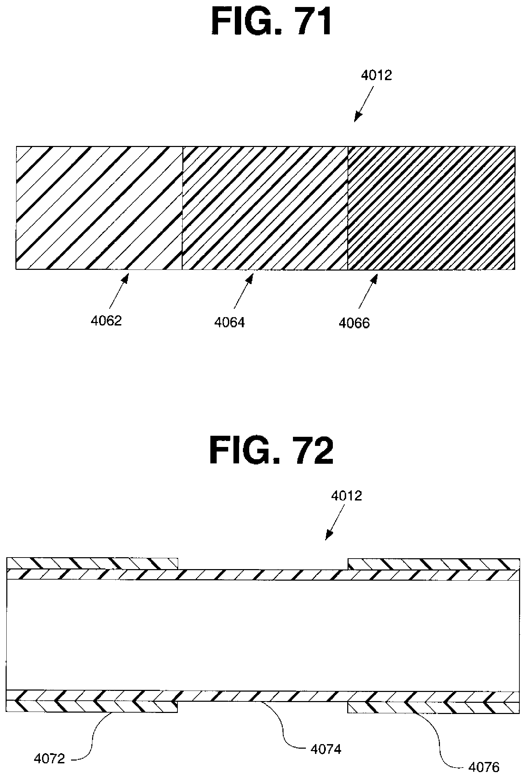

FIG. 71 illustrates a first embodiment of a shape memory material having at least two glass transition temperatures. Fig. 71 illustrates the shape memory material 4012 having at least two glass transition temperatures. In the depicted embodiment, the shape memory material 4012 has three different sections 4062, 4064, 4066, each with a different glass transition temperature. The different glass transition temperatures allow the shape memory material to be designed such that the sections expand in a certain order. For example, sections 4062 and 4066 may be expanded before section 4064 or vice versa. Although three sections are shown, any number of sections are possible. By controlling how the shape memory material expands, adequate expansion on each side of a fracture site can be ensured and desired placement of the expanded shape memory material can be achieved.

FIG. 72 illustrates a second embodiment of a shape memory material having at least two glass transition temperatures. Fig. 72 illustrates the shape memory material 4012 having at least two glass transition temperatures. In the depicted embodiment, the shape memory material 4012 has three different sections 4072, 4074, 4076, each with a different glass transition temperature. The second section 4074 extends the entire length of the shape memory material. The first section 4072 and the third section 4076 cover the end portions of the second section. By controlling how the shape memory material expands, adequate expansion on each side of a fracture site can be ensured and desired placement of the expanded shape memory material can be achieved.

The embodiment depicted in FIG. 72 may be achieved by masking off sections of the shape memory material and treating the unmasked sections with a plasticizer or solvent. This may provide a shape memory material with different sections having different glass transition temperatures.

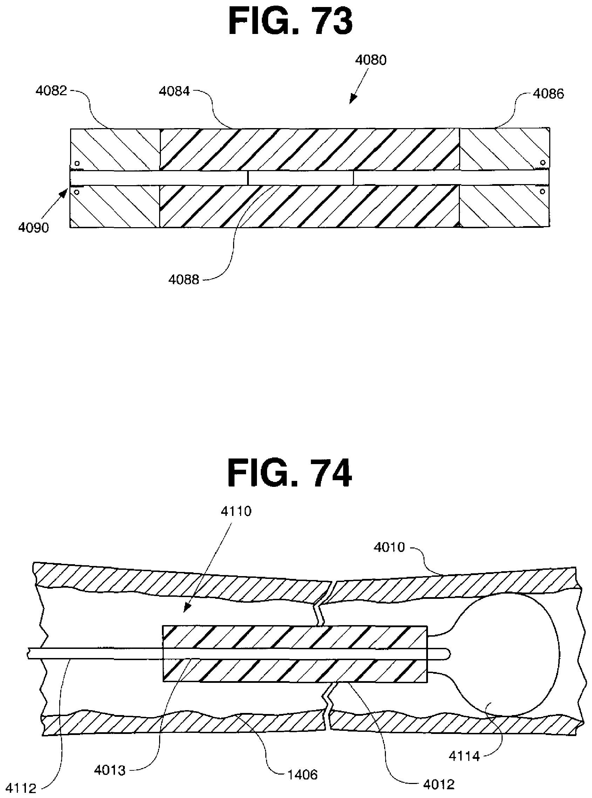

FIG. 73 illustrates an implant assembly 4080. The implant assembly 4080 includes a shape memory material 4084, a first cap 4082, and a second cap 4086. The caps 4082, 4086 are made of a biocompatible material. In the depicted embodiment, the caps 4082, 4086 are made of titanium. In some embodiments, the shape memory material 4084 is cannulated and a rod or tube 4088 connects the caps 4082, 4086. The caps 4082, 4086 may or may not include a threaded hole 4090. The threaded hole may be used to install or remove the implant assembly.

In some embodiments, the caps 4082, 4086 may be adapted to move toward one another to apply pressure on the shape memory polymer. As an example, each cap may be spring loaded to apply a biasing force on the shape memory material as it expands. As another example, an operator may apply a biasing force to the shape memory material using an instrument.

FIG. 74 illustrates a first instrument for placement of the shape memory material. FIG. 74 illustrates bone 4010, shape memory material 4012, cannulation 4013, intramedullary cavity 4016, and a first instrument 4110. The first instrument includes a heating device 4112 and a balloon 4114. The balloon 4114 is deployed and the heating device 4112 is energized. The balloon 4114 may be used to prevent the shape memory material from shifting to one side as it expands. Alternatively, a surgeon may use the balloon 4114 to translate the shape memory material as it expands. After expansion and placement, the balloon is deflated and removed through the cannulation 4013.

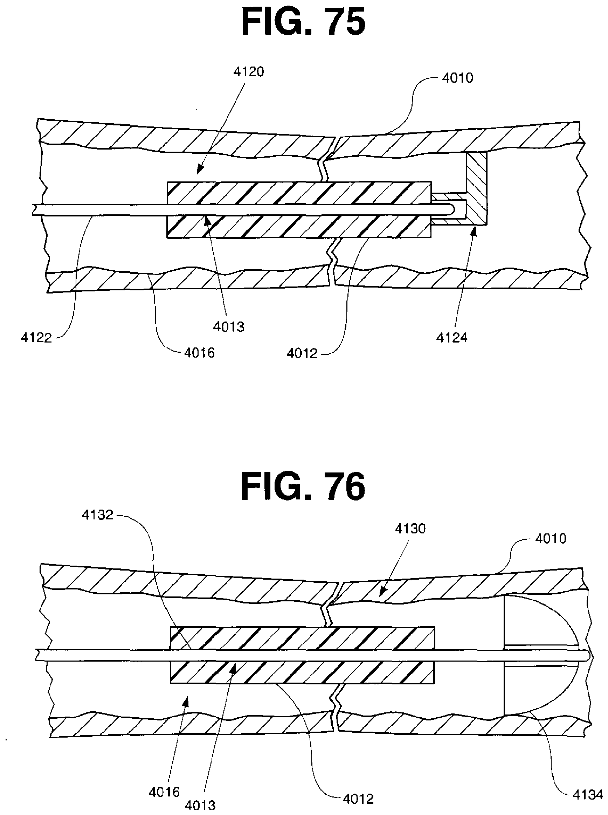

FIG. 75 illustrates a second instrument for placement of the shape memory material. FIG. 75 illustrates bone 4010, shape memory material 4012, cannulation 4013, intramedullary cavity 4016, and a second instrument 4120. The second instrument 4120 includes a heating device 4122 and a deployable anchor 4124. The deployable anchor 4124 is deployed and the heating device 4112 is energized. The deployable anchor 4124 may be used to prevent the shape memory material from shifting to one side as it expands. Alternatively, a surgeon may use the deployable anchor 4124 to translate the shape memory material as it expands. After expansion and placement, the deployable anchor 4124 is contracted and removed through the cannulation 4013.

FIG. 76 illustrates a third instrument for placement of the shape memory material. FIG. 76 illustrates bone 4010, shape memory material 4012, cannulation 4013, intramedullary cavity 4016, and a second instrument 4130. The third instrument 4130 includes a heating device 4132 and a second shape memory material 4134. The shape memory material 4012, the second shape memory material 4134, and the heating device 4132 are placed within the intramedullary cavity 4016. The second shape memory material 4134 is deployed and thereafter the shape memory material 4012 is energized and expanded. The second shape memory material 4134 may be used to prevent the shape memory material 4012 from shifting to one side as it expands.

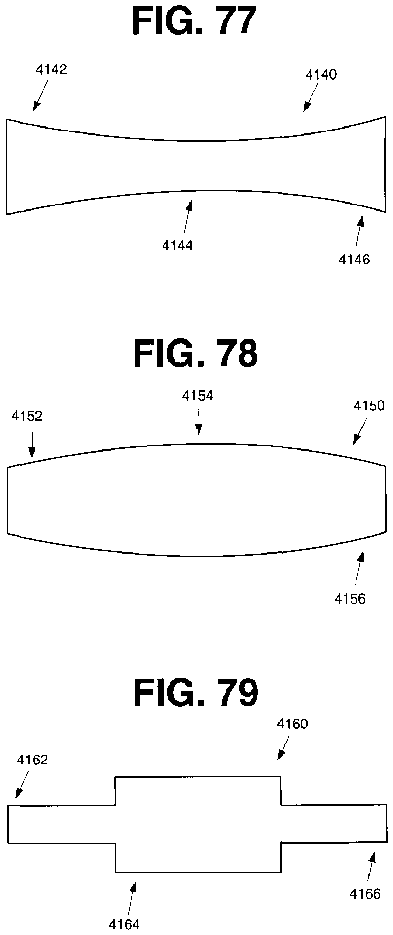

FIGS. 77-81 illustrate various shapes of the shape memory material. The shape may be selected to encourage the shape memory material to expand and obtain fixation in one area before another. For example, the material may be shaped such that the thinner portion expands before the thicker portion. Thus the overall shape of the shape memory material allows for adequate expansion on each side of a fracture site and may be used to achieve desired placement of the expanded shape memory material. Although three portions are shown in each of the following embodiments, any number of portions or sections may be used.

FIG. 77 illustrates a shape memory material 4140 in a first embodiment. In this embodiment, the shape memory material 4140 has a first end portion 4142, a middle portion 4144, and a second end portion 4146. In the depicted embodiment, the ends 4142, 4146 are thicker than the middle portion 4144. In the depicted embodiment, there is provided a smooth arcuate taper towards the middle but a sharp taper may equally be used.

FIG. 78 illustrates a shape memory material 4150 in a second embodiment. In this embodiment, the shape memory material 4150 has a first end portion 4152, a middle portion 4154, and a second end portion 4156. In the depicted embodiment, the ends 4152, 4156 are thinner than the middle portion 4154. Although a smooth arcuate transition is shown, a sharp taper may equally be used.

FIG. 79 illustrates a shape memory material 4160 in a third embodiment. In this embodiment, the shape memory material 4160 has a first end portion 4162, a middle portion 4164, and a second end portion 4166. In the depicted embodiment, the ends 4162, 4166 have a smaller diameter than the middle portion 4164. Although portions 4162, 4164, 4166 are depicted as cylindrical, other shapes may equally be used.

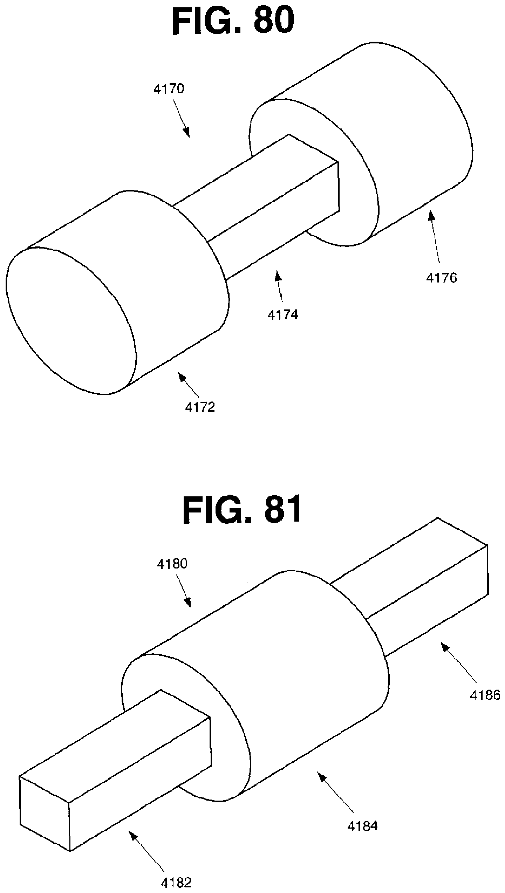

FIG. 80 illustrates a shape memory material 4170 in a fourth embodiment. In this embodiment, the shape memory material 4170 has a first end portion 4172, a middle portion 4174, and a second end portion 4176. The first end portion 4172 and the second end portion 4176 have a first shape and the middle portion 4174 has a second shape. In the depicted embodiment, the first end portion 4172 and the second end portion 4176 are cylindrical, and the middle portion 4174 is square. Other shapes than those depicted may be used.

FIG. 81 illustrates a shape memory material 4180 in a fifth embodiment. In this embodiment, the shape memory material 4180 has a first end portion 4182, a middle portion 4184, and a second end portion 4186. The first end portion 4182 and the second end portion 4186 have a first shape and the middle portion 4184 has a second shape. In the depicted embodiment, the first end portion 4172 and the second end portion 4176 are square, and the middle portion 4174 is cylindrical. Other shapes than those depicted may be used.

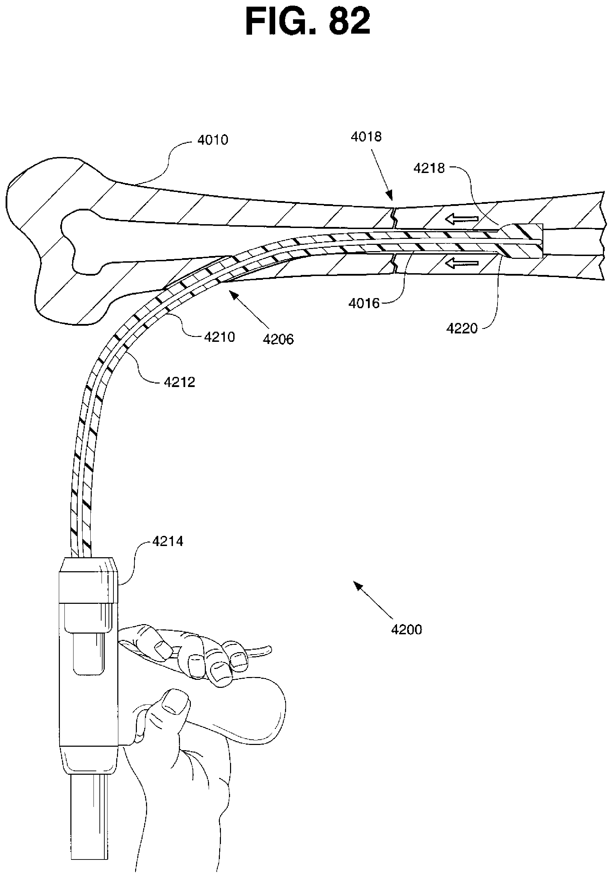

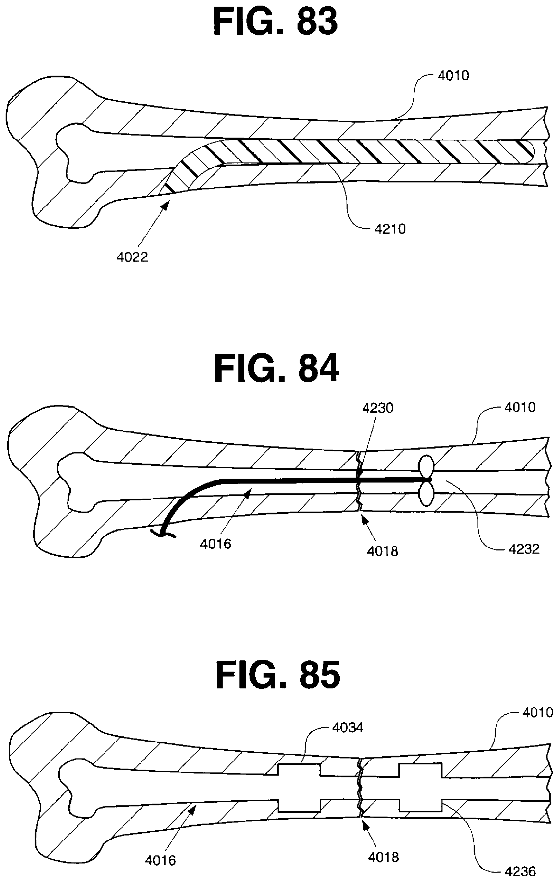

FIG. 68 illustrates the cut-to-length shape memory material, and FIG. 82 illustrates a fourth instrument for placement of the shape memory material. FIG. 82 illustrates the bone 4010, the intramedullary cavity 4016, and a fourth instrument 4200 for installing the cut-to-length shape memory material. The fourth instrument 4200 includes cut-to-length shape memory material 4210, a heating device 4212, and an activator 4214. In some embodiments, the cut-to-length shape memory material 4210 may be flexible. The heating device 4212 includes a tip portion 4220. In some embodiments, the heating device 4212 may be flexible. The heating device 4212 provides energy, such as heat, at the tip portion 4220. The cut-to-length shape memory material 4210 is cannulated and the heating device is located within the cannulation. In this embodiment, an opening 4206 is created into a portion of the bone 4010. The cut-to-length shape memory material 4210 is inserted into the opening 4206 and fed into the intramedullary cavity until a distal end portion 4218 is adequately placed relative to the fracture site 4018. Thereafter, the cut-to-length shape memory material 4210 is energized to allow for adequate expansion on each side of the fracture site 4018 to engage the endosteal surface and/or cancellous bone. The shape memory material 4210 is activated by triggering the activator 4214, which sends energy to the tip portion 4220. As the tip portion 4220 heats up, the shape memory material expands. An operator slowly pulls on the activator 4214 to slowly withdraw the tip portion 4220 through the cannulation. As the tip portion 4220 travels through the cannulation, it heats up the surrounding shape memory material 4210. This continues until the tip portion is removed from the bone 4010. Finally, the excess shape memory material is removed at the opening 4206. FIG. 83 illustrates the installed shape memory material 4210 after expansion. The area 4222 indicates where the excess shape memory material has been removed.

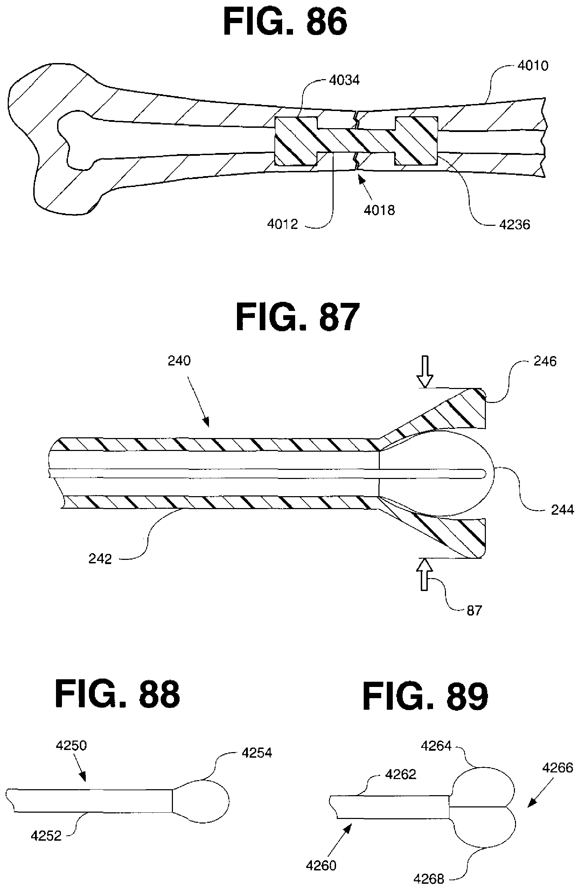

FIG. 84 illustrates a shapable reamer 4230, bone 4010, fracture site 4018, and the intramedullary cavity 4016. The shapable reamer 4230 has deployable blades to selectively ream portions of the intramedullary cavity 4016. FIG. 85 illustrates bone 4010 after reaming using the shapable reamer 4230. In the depicted embodiment, there is a first reamed section 4234 and a second reamed section 4236. Those having ordinary skill in the art would understand that any number of reamed sections or voids may be achieved. The reamed sections may have radius from about one to about eight millimeters greater than the approximate radius of the shape memory material. In the depicted embodiment, the reamed sections have a radius of about four millimeters larger than the approximate radius of the shape memory material. FIG. 86 illustrates the shape memory material 4012 as installed in the reamed intramedullary cavity 4016. The reamed sections 4234, 4236 allow for adequate expansion on each side of a fracture site and achieve desired placement of the expanded shape memory material. Placement of the shape memory material in the reamed sections provides axial stability.

The invention further includes a method of installing a shape memory polymer. First, the fracture site is located, such as by using a C-arm or X-ray machine. Second, a first void is reamed on a first side of the fracture site. Second, a second void is reamed on a second side of the fracture site. Third, a shape memory material is inserted into the intramedullary cavity and placed relative to the first and second void. Fourth, energy is applied to the shape memory polymer such that it expands at least into the first and second void.

There are many ways of achieving the shapable reamer 4230. FIG. 87 illustrates merely one exemplary embodiment of the shapable reamer. FIG. 87 illustrates a shapable reamer 4240. The shapable reamer 4240 includes a shaft 4242, a balloon 4244, and deployable reamer blades 4246. Initially, the balloon is deflated such that the blades 4246 may be placed within an intramedullary cavity. When located at the desired location, the balloon is inflated to deploy the blades 4246. Thereafter, the shaft 4246 is rotated to ream and may be translated to create a void.

FIG. 88 illustrates a first embodiment of a balloon compression device. In some applications, it may not be necessary to ream a void within the wall of the intramedullary cavity, and the bone may be merely compressed to achieve a void. FIG. 88 illustrates a compressive device 4250. The compressive device 4250 includes a shaft 4252 and a balloon 4254. The balloon 4254 is initially deflated. The balloon 4254 is placed at the desired void location and inflated. The pressure from the balloon 4254 presses against the walls and creates a void.

FIG. 89 illustrates a second embodiment of a balloon compression device. FIG. 89 illustrates a compressive device 4260. The compressive device 4260 includes a shaft 4262 and a balloon 4266. In the depicted embodiment, the balloon 4266 has a first half or chamber 4264 and a second half or chamber 4268. The balloon 4266 is initially deflated. The balloon 4266 is placed at the desired void location and inflated. The pressure from the balloon 4266 presses against the walls and creates a void. The halves 4264, 4268 may be selectively pressurized to control the position and/or direction in which the void is created. Although two halves are shown, the balloon 4266 may have any number of chambers.

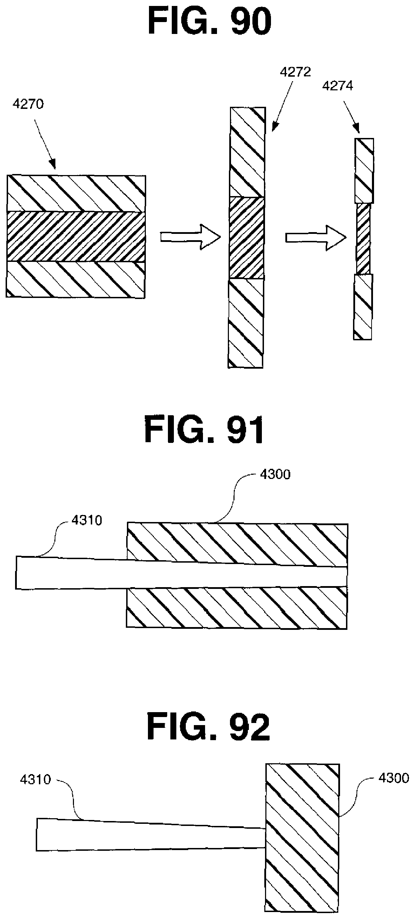

FIG. 90 illustrates a method for manufacturing a shape memory material having at least two glass transition temperatures to achieve a shape memory material having multiple activation temperatures. In step 4270, at least two different materials are layered to produce a billet. In the depicted embodiment, there are three materials, each having a different glass transition temperature. In step 4272, the billet is die drawn to orientate the polymer. In optional step 4274, the material is machined to achieve a final shape.

FIGS. 91-92 illustrate a tapered heater in a first embodiment. FIGS. 91-92 illustrate a shape memory material 4300 and a tapered heating element 4310. The heating element is tapered such that the shape memory material shifts to one side of the taper as it expands. In the depicted embodiment, the shape memory material 4300 shifts towards the small end as it expands and thereafter the heater can be removed.

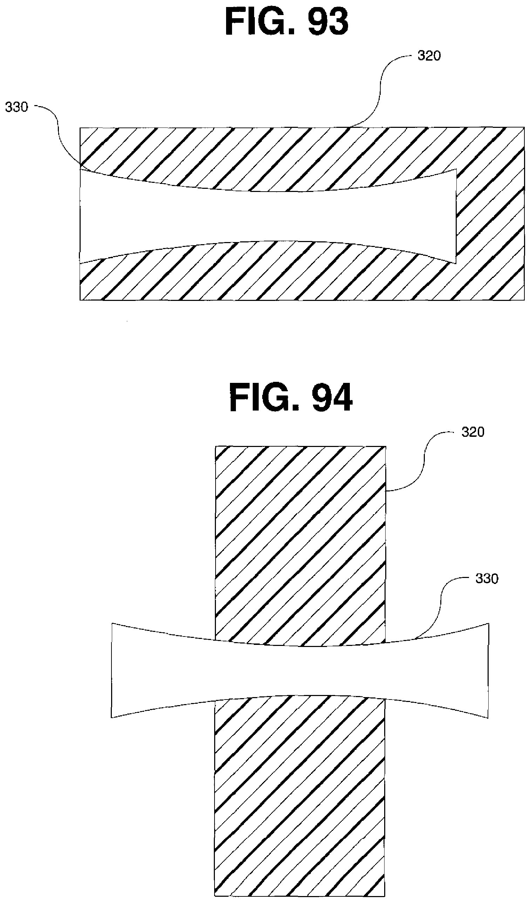

FIGS. 93-94 illustrate a tapered heater in a second embodiment. FIGS. 93-94 illustrate a shape memory material 4320 and a tapered heating element 4330. The heating element is tapered such that the shape memory material shifts to towards the center of the heating element as it expands. In the depicted embodiment, heating element 4330 can be removed while the shape memory material is still pliable.

As stated throughout this disclosure, internal fixation devices are used for fracture reduction, fixation or stabilization, and compression. For devices such as intramedullary nails, rods, and pins, fracture reduction and stabilization may occur via a method according to the following steps: creation of an entry portal at a location along the bone, provisional reduction of a fracture via the use of a reducer or other tool known to one of ordinary skill in the art for reducing fractures, insertion of the internal fixation device through the entry portal and placement of the device across the fracture, fixation of one side of the fracture by insertion of at least one fastener through the device or, as described above, expansion of a shape memory polymer material, reduction of the fracture via the application of pressure on the device or on the other side of the fracture that has not been fixated, and fixation of the other side of the fracture by insertion of at least one fastener through the device or, as described above, expansion of a shape memory polymer material. It is within the scope of this disclosure that the fixation and reduction steps may occur in a different order. For example, both sides of the fracture may be fixated before the fracture is reduced. This is especially true through, as described above, the use of a device that can compress the fracture via the use of an expandable polymer material.

For a device, such as a bone plate, fracture reduction and stabilization may occur via a method according to the following steps: reduction of the fracture, placement of a plate across the fracture via the use of an instrument or provisional fixation device, such as a forceps pin, to hold the plate to the bone while the plate is being fixated to the bone, placement of at least one non-locking or locking fastener through a hole in the plate and a hole on one side of the fracture, placement of at least one nonlocking or locking fastener through a hole in the plate and a hole on another side of the fracture and compression of the fracture either manually, via the use of compression screws, or with a device that uses expandable polymer material, as described above. It is within the scope of this disclosure that the holes may be made in the plate and the bone after the plate is placed across the fracture. The holes may be created through the use of a drill, a tap, a broach, or another instrument known to one of ordinary skill in the art for creating holes in the plate and bone. It is also within the scope of this disclosure that the fasteners may be fasteners that lock to the plate via the use of expandable material on either the head of the fastener or on the inner wall of the plate or bone holes, as described above. Also, multiple fasteners, used on one or both sides of the fracture, may be used to fixate the plate to the bone. It is within the scope of this disclosure that the fixation and reduction steps may occur in a different order. For example, both sides of the fracture may be fixated before the fracture is reduced. This is especially true through, as described above, the use of a device that can compress the fracture via the use of an expandable polymer material.

When the polymer material includes alternating segments of a shape memory polymer material and a non-shape memory polymer material, each shape memory segment can be individually provided with energy at separate time intervals in order to gradually cause straightening, bending, shortening, or lengthening of the material. For example, a first segment of shape memory polymer material can be activated to expand and shorten. This causes a first length of the material attached to the device to shorten. After a period of time, a second segment of shape memory polymer is activated to cause further shortening of material. This is beneficial for treatments requiring long-term remodeling, such as scoliosis or other deformities.

The present disclosure is directed to the use of electrical and thermal energy sources to heat the polymer material and deform it. However, the polymer material could be deformed via other methods known to those of ordinary skill in the art, including, but not limited to the use of force, or mechanical energy, a solvent, and /or a magnetic field. Any suitable force that can be applied either preoperatively or intra-operatively can be used. One example includes the use of ultrasonic devices, which can deform the polymer material with minimal heat generation. Solvents that could be used include organic-based solvents and aqueous-based solvents, including body fluids. Care should be taken that the selected solvent is not contra indicated for the patient, particularly when the solvent is used intra-operatively. The choice of solvents will also be selected based upon the material to be deformed. Examples of solvents that can be used to deform the polymer material include alcohols, glycols, glycol ethers, oils, fatty acids, acetates, acetylenes, ketones, aromatic hydrocarbon solvents, and chlorinated solvents. Finally, the polymer material could include magnetic particles and deformation could be initiated by inductive heating of the magnetic particles through the use of a magnetic field.

## Claims

1. An internal fixation device for use in conjunction with a bone fracture site in a mammal, the internal fixation device comprising:
at least one interface portion and at least one sleeve formed of polymer material, wherein the polymer material includes shape memory qualities, said sleeve defining a channel with said interface portion positioned within said channel; and
means for allowing adequate expansion of the polymer material;
the means for allowing adequate expansion of the polymer material comprising means for heating the polymer material, thereby to expand the sleeve radially and fixate the fixation device to the bone, and to shrink the sleeve axially to at least partially reduce the bone fracture site.

2. The internal fixation device of claim 1 further comprising at least one feature on an outer surface of said sleeve, wherein the at least one feature provides multiple contact points to increase friction between the polymer material and the bone.

3. The internal fixation device of claim 2 wherein the at least one feature comprises at least one protrusion coupled to said sleeve.

4. The internal fixation device of claim 3 wherein the at least one protrusion comprises a plurality of serrations located on an outside surface of said protrusion.

5. The internal fixation device of claims 3 or 4 wherein the at least one protrusion is selected from a group consisting essentially of a metal material, a non-metal material, a polymer material, and combinations thereof.

6. The internal fixation device of any one of claims 2 to 5 wherein the at least one feature comprises a particulate material within or on an outer surface of said sleeve.

7. The internal fixation device of claim 6 wherein the particulate material comprises a ceramic material.

8. The internal fixation device of any one of the preceding claims wherein the polymer material includes a resorbable or a non-resorbable polymer material.

9. The internal fixation device of any one of the preceding claims wherein said sleeve has a circular outer cross section and said channel has a non-circular inner cross section.

10. The internal fixation device of any one of the preceding claims wherein said interface portion comprises a shaft portion of an intramedullary nail.

11. The internal fixation device of any one of the preceding claims wherein heating the polymer material comprises providing thermal energy to the polymer material.

## Patentansprüche

1. Eine interne Fixierungsvorrichtung zur Verwendung zusammen mit einer Knochenbruchstelle in einem Säugetier, wobei die interne Fixierungsvorrichtung Folgendes beinhaltet:
mindestens einen Kontaktabschnitt und mindestens eine aus Polymermaterial gebildete Manschette, wobei das Polymermaterial Formgedächtniseigenschaften umfasst, wobei die Manschette einen Kanal definiert und der Kontaktabschnitt innerhalb des Kanals positioniert ist; und
ein Mittel, um eine geeignete Expansion des Polymermaterials zu erlauben;
wobei das Mittel, um eine geeignete Expansion des Polymermaterials zu erlauben, ein Mittel zum Erwärmen des Polymermaterials beinhaltet, wodurch die Manschette radial expandiert und die Fixierungsvorrichtung an dem Knochen fixiert wird und die Manschette axial schrumpft, um die Knochenbruchstelle mindestens teilweise zu verkleinern.

2. Interne Fixierungsvorrichtung gemäß Anspruch 1, ferner beinhaltend mindestens ein Merkmal auf einer Außenoberfläche der Manschette, wobei das mindestens eine Merkmal mehrere Berührungspunkte bereitstellt, um die Reibung zwischen dem Polymermaterial und dem Knochen zu verstärken.

3. Interne Fixierungsvorrichtung gemäß Anspruch 2, wobei das mindestens eine Merkmal mindestens einen an die Manschette gekoppelten Vorsprung beinhaltet.

4. Interne Fixierungsvorrichtung gemäß Anspruch 3, wobei der mindestens eine Vorsprung eine Vielzahl von Verzahnungen beinhaltet, die sich an einer äußeren Oberfläche des Vorsprungs befinden.

5. Interne Fixierungsvorrichtung gemäß Anspruch 3 oder 4, wobei der mindestens eine Vorsprung aus einer Gruppe ausgewählt ist, die im Wesentlichen aus einem Metallmaterial, einem Nichtmetallmaterial, einem Polymermaterial und Kombinationen davon besteht.

6. Interne Fixierungsvorrichtung gemäß einem der Ansprüche 2 bis 5, wobei das mindestens eine Merkmal ein partikelförmiges Material innerhalb oder an einer Außenoberfläche der Manschette beinhaltet.

7. Interne Fixierungsvorrichtung gemäß Anspruch 6, wobei das partikelförmige Material ein Keramikmaterial beinhaltet.

8. Interne Fixierungsvorrichtung gemäß einem der vorhergehenden Ansprüche, wobei das Polymermaterial ein resorbierbares oder nicht resorbierbares Polymermaterial umfasst.

9. Interne Fixierungsvorrichtung gemäß einem der vorhergehenden Ansprüche, wobei die Manschette einen kreisförmigen Außenquerschnitt aufweist und der Kanal einen nicht kreisförmigen Innenquerschnitt aufweist.

10. Interne Fixierungsvorrichtung gemäß einem der vorhergehenden Ansprüche, wobei der Kontaktabschnitt einen Schaftabschnitt eines intramedullären Nagels beinhaltet.

11. Interne Fixierungsvorrichtung gemäß einem der vorhergehenden Ansprüche, wobei das Erwärmen des Polymermaterials das Bereitstellen von thermischer Energie für das Polymermaterial beinhaltet.

## Revendications

1. Un dispositif de fixation interne pour une utilisation en association avec un site de fracture osseuse chez un mammifère, le dispositif de fixation interne comprenant :
au moins une portion d'interface et au moins un manchon formé de matériau polymère, dans lequel le matériau polymère inclut des qualités de mémoire de forme, ledit manchon définissant un canal avec ladite portion d'interface positionnée au sein dudit canal ; et
un moyen pour permettre un déploiement adéquat du matériau polymère ;
le moyen pour permettre un déploiement adéquat du matériau polymère comprenant un moyen pour chauffer le matériau polymère, pour ainsi déployer le manchon de manière radiale et fixer le dispositif de fixation à l'os, et pour rétrécir le manchon de manière axiale pour réduire au moins partiellement le site de fracture osseuse.

2. Le dispositif de fixation interne de la revendication 1 comprenant en outre au moins un élément sur une surface externe dudit manchon, dans lequel l'au moins un élément fournit plusieurs points de contact pour augmenter le frottement entre le matériau polymère et l'os.

3. Le dispositif de fixation interne de la revendication 2 dans lequel l'au moins un élément comprend au moins une saillie couplée audit manchon.

4. Le dispositif de fixation interne de la revendication 3 dans lequel l'au moins une saillie comprend une pluralité de cannelures situées sur une surface extérieure de ladite saillie.

5. Le dispositif de fixation interne des revendications 3 ou 4 dans lequel l'au moins une saillie est sélectionnée dans un groupe constitué essentiellement d'un matériau métallique, d'un matériau non métallique, d'un matériau polymère, et de combinaisons de ceux-ci.

6. Le dispositif de fixation interne de n'importe laquelle des revendications 2 à 5 dans lequel l'au moins un élément comprend un matériau particulaire dans ou sur une surface externe dudit manchon.

7. Le dispositif de fixation interne de la revendication 6 dans lequel le matériau particulaire comprend un matériau céramique.

8. Le dispositif de fixation interne de n'importe laquelle des revendications précédentes dans lequel le matériau polymère inclut un matériau polymère résorbable ou non résorbable.

9. Le dispositif de fixation interne de n'importe laquelle des revendications précédentes dans lequel ledit manchon a une section transversale externe circulaire et ledit canal a une section transversale interne non circulaire.

10. Le dispositif de fixation interne de n'importe laquelle des revendications précédentes dans lequel ladite portion d'interface comprend une portion de tige d'un clou intramédullaire.

11. Le dispositif de fixation interne de n'importe laquelle des revendications précédentes dans lequel le chauffage du matériau polymère comprend la fourniture d'énergie thermique au matériau polymère.
